# EUROPEAN PATENT APPLICATION

(11) **EP 2 671 596 A1**
(43) Date of publication of application: **11.12.2013**
(21) Application number: 12742635.1
(22) Date of filing: 31.01.2012
(51) Int. Cl.: A61K 47/40, A23L 1/30, A23L 1/303, A61K 8/73, A61K 9/08, A61K 31/05, A61K 31/07, A61K 31/12, A61K 31/122, A61K 31/192, A61K 31/216, A61K 31/352, A61K 31/381, A61K 35/64, A61K 36/73, A61K 47/14, A61K 47/26, A61K 47/28, A61K 47/34, A61K 47/36

(54) **PROCESS FOR PRODUCING AQUEOUS SOLUTION CONTAINING FAT-SOLUBLE SUBSTANCE**

(30) Priority: 31.01.2011 JP 2011019112; 28.12.2011 JP 2011287729
(71) Applicant: Wacker Chemie AG, 81737 München (DE)
(72) Inventor: TERAO, Keiji, Kobe-shi, Hyogo 650-0047 (JP); FUKUMI, Hiroshi, Kobe-shi, Hyogo 650-0047 (JP); NAKATA, Daisuke, Kobe-shi, Hyogo 650-0047 (JP); UEKAJI, Yukiko, Kobe-shi, Hyogo 650-0047 (JP); JO, Ayako, Kobe-shi, Hyogo 650-0047 (JP)
(74) Representative: Potten, Holger
(86) International application number: PCT/JP2012/052126
(87) International publication number: WO 2012/105546

(57) **Abstract**

The purpose of the present invention is to provide a process for producing an aqueous solution containing a fat-soluble substance. A combination of a super dissolution aid, cyclodextrin and a fat-soluble substance or a combination of the super dis-solution aid and a cyclodextrin complex of the fat-soluble substance is used for dissolving the fat-soluble substance, whereby it becomes possible to provide an aqueous solution containing the fat-soluble substance at a high concentration and an aqueous solution having improved transdermal absorbability of the fat-soluble substance contained therein.

## Description

### Technical Field

The present invention relates to a method for producing an aqueous solution containing a fat-soluble substance. More specifically, the present invention relates to a method for the production of an aqueous solution which is capable of containing a fat-soluble substance at high concentration and which features high solubility for fat-soluble substances by combining and using a super solubilizer and cyclodextrin (hereafter sometimes referred to as "CD") upon dissolution of the fat-soluble substance. Furthermore, the present invention relates to an aqueous solution which contains a fat-soluble substance obtained via this method as well as an aqueous solution which is characterized by high absorbance of the fat-soluble substance by the skin obtained via this method.

### Background Art

Fat-soluble substances such as polyphenols, including curcumin; isoflavanoids and flavanoids, including nobiletin; tocopherols, including tocotrienol; retinols, including retinol; menaquinones, including phylloquinone; carotenoids, including astaxanthin; cinnamic acid derivatives, including caffeic acid phenethyl ester (hereafter sometimes referred to as "CAPE"); diterpenes which include as an active ingredient a cinnamic acid derivative, including propolis and phytol; terpenes, including squalene; coenzyme Q10 (hereafter sometimes referred to as "CoQ10") and α-lipoic acid are currently used in a variety of different products, such as cosmetics and foods, as active ingredients which are effective in promoting skin whitening and anti-aging.
However, because these substances are fat-soluble, they cannot be incorporated into water in high concentration, are difficult to handle in the manufacture of cosmetics and foods and cannot be fully utilized. Therefore, the provision of a method to solubilize these fat-soluble substances so that they can be used in aqueous solution is desired.

For example, a method for increasing the solubility of the fat-soluble substance curcumin in water by combining it with CD has been disclosed. However, the concentration of curcumin in aqueous solution obtained using this method is low at 14 µg / mL (for example, see Patent Document 1) or 34.2 µg / mL (for example, see Patent Document 2) and curcumin cannot be said to be sufficiently solubilized.
Furthermore, according to a method found by the inventors of the present invention (Patent Document 2, Table 4), when α-cyclodextrin (hereafter sometimes referred to as "αCD") is combined with curcumin, it is possible to obtain an aqueous solution containing a high concentration (269 µg / mL, 262 µg / mL, or 237 µg / mL) of curcumin. However, because this concentration was measured using an absorption spectrometer, it also includes impurities other than curcumin, and does not constitute a concentration obtained by measuring only the concentration of curcumin. Additionally, given that the maximum value of the concentration of the standard solution which was used as a reference was 100 µg / mL (Patent Document 2, Table 3), the concentration is outside the measurement range of the standard and is likely unreliable.
Accordingly, the inventors of the present invention prepared an aqueous solution of curcumin comparable to that shown in Patent Document 2, Table 4, and performed follow-up measurements of the concentration of only curcumin in solution using HPLC under the same conditions as those used for the present invention. The results showed that the concentration of curcumin was low at 1.47 µg / mL, 3.63 µg / mL or 8.29 µg / mL when the content of αCD was 1%, 2.5% or 5% respectively. Therefore, the method described in Patent Document 2 cannot be regarded as a method by which an aqueous solution containing curcumin in high concentration is obtained.

Methods in which an aqueous solution of glycerin fatty acid ester, sucrose fatty acid ester, phospholipid, saponin or polysorbate (polyoxyethylene sorbitan fatty acid) is mixed with a surfactant after heating and melting a fat-soluble substance such as lycopene, CoQ10 or β-carotene (for example, Patent Document 3) have been disclosed as methods for the solubilization of fat-soluble substances other than curcumin. Further, a method in which licorice oil extract, saponin and a solubilizer are mixed together in a certain mass ratio to produce a solubilized composition has been disclosed, and CD is proposed as one possible solubilizer (for example, see Patent Document 4). Furthermore, a method to produce a solubilized composition by inclusion of CD has been proposed for nobiletin as well, but stirring over a long period of time is required (for example, Patent Document 5). Moreover, none of these methods achieve production of an aqueous solution containing a fat-soluble substance at high concentrations.

Among available fat-soluble substances, CoQ10 in particular has drawn attention as a useful ingredient for achieving anti-aging and skin-whitening effects and a number of different cosmetics and cutaneous external preparations containing CoQ10 have been developed.
For example, Patent Document 6, discloses a CoQ10-containing cosmetic composition containing a polyhydric alcohol and a surfactant containing a predetermined minimum amount of CoQ10 and polyglyceryl fatty acid ester.
In Patent Document 6, the authors verified the percutaneous absorption of the CoQ10-containing cosmetic composition using a three-dimensional cultured skin model. However their results showed that the degree of incorporation of CoQ10 into the three-dimensional cultured skin model was only 2-4 times greater when the composition was adjusted to include a specific polyglyceryl fatty acid ester versus when polyglyceryl fatty acid ester was not included.

Furthermore, Patent Document 7 discloses a cutaneous external preparation containing ubiquinones such as CoQ10, glycol ethers and phospholipids and transdermal absorption testing using hairless mice was performed. However, their results also showed that the amount of CoQ10 accumulated in the skin was only approximately 3.6 - 8.4 times higher when the composition was modified to include ethoxydiglycol versus compositions modified to include glycerin.
Further, Patent Document 8 discloses a type of cationic polymer nanocapsule used to increase dermal absorption which incorporates captured CoQ10, and the degree of dermal absorption was measured using a marmot skin model. However, the degree of skin absorption was only increased by 1.5 - 5.9 times relative to non-cationic polymer nanocapsules.
Thus, conventional methods cannot be said to obtain a material which significantly incorporates CoQ10 into the skin, and the provision of a method which more effectively solubilizes CoQ10 for use in cosmetics and foods is desired.

### Prior Art Documents

### Patent Documents

Patent Document 1: Publication No. JP 2005-328839
Patent Document 2: Publication No. JP 2006-111534
Patent Document 3: Publication No. JP 2006-030850
Patent Document 4: Publication No. JP 2004-065128
Patent Document 5: Publication No. JP 2008-074723
Patent Document 6: Publication No. JP 2008-106012
Patent Document 7: Publication No. JP 2006-213699
Patent Document 8: Publication No. JP 2009-514811

### Summary of the Invention

### Problem Which the Invention Attempts to Solve

The present invention attempts to provide a method for the production of an aqueous solution which can incorporate a fat-soluble substance at high concentrations. Furthermore, the present invention attempts to provide an aqueous solution which contains a fat-soluble substance at high concentration obtained via this method as well as an aqueous solution which is characterized by high absorbance of the fat-soluble substance by the skin obtained via this method.

### Method for Solving the Problem

In order to solve the aforementioned issue, the inventors of the present invention have conducted intensive research and, after determining that it is possible to produce an aqueous solution capable of incorporating fat-soluble substances at high concentration using production methods which include Process A or B, below, upon dissolution of the fat-soluble substance, completed the present invention.
A. A process in which a super solubilizer, cyclodextrin and the aforementioned fat-soluble substance are added to water
B. A process in which a super solubilizer and cyclodextrin complex containing a fat-soluble substance are added to water
Furthermore, the inventors of the present invention have determined that, using this method, it is possible to obtain an aqueous solution characterized in that the dermal absorbance of the fat-soluble substance is increased.

That is, the present invention relates to a method for producing an aqueous solution containing a fat-soluble substance as stated in (1) - (20) below. Furthermore, the present invention relates to an aqueous solution which contains a fat-soluble substance obtained via this method as well as an aqueous solution which is characterized by high absorbance of the fat-soluble substance by the skin obtained via this method.
(1) A method for the production of an aqueous solution containing one or more of the following fat-soluble substances: polyphenols, flavonoids, tocopherols, retinols, menaquinones, carotenoids, propolis or cinnamic acid derivatives, diterpenes, terpenes, coenzyme Q10 or α-lipoic acid, which includes Process A or B below.
   A. A process in which one or more of the super solubilizers listed in 1) - 9), cyclodextrin and the aforementioned fat-soluble substance are added to water
   B. A process in which one or more of the super solubilizers listed in 1) - 9) and a cyclodextrin complex containing a fat-soluble substance are added to water
      1) An alkali salt or bile acid selected from any one of the following: taurocholate, glycocholate, cholic acid, lithocholic acid or deoxycholic acid
      2) Glycosides of polyphenols or hesperidin
      3) An emulsifier selected from any one of the following: glycerin fatty acid ester, sorbitan fatty acid ester, propylene glycol fatty acid ester, or sucrose fatty acid ester
      4) A lecithin agent selected from any one of the following: enzymatically decomposed lecithin, lecithin derived from soybean or lecithin derived from egg yolk
      5) A saponin selected from any one of the following: glycyrrhizic acid or an alkali salt thereof, soybean saponin or quillaja saponin
      6) A casein or alkali salt thereof
      7) A polysorbate (fatty acid polyoxyethylene sorbitan)
      8) A cellulose or alkali salt thereof selected from any one of the following: methylcellulose, carboxymethyl cellulose or hydroxyethyl cellulose
      9) A polysaccharide thickener selected from any one of the following: pectin, carrageenan or xanthan gum
(2) The method specified in (1) above in which the cyclodextrin employed is either α-cyclodextrin, β-cyclodextrin, γ-cyclodextrin or branched cyclodextrin.
(3) The method specified in (1) or (2) above in which the fat-soluble substance is one of those listed in 1) - 11) below.
   1) A polyphenol selected from any one of the following: curcumin, rose polyphenol, ferulic acid, quercetin or cacao mass
   2) A flavanoid selected from any one of the following: isoflavone, isoflavone aglycone, flavanone, anthocyanidin, catechin, chalcone or nobiletin
   3) A tocopherol selected from any one of the following: tocotrienol, γ-tocotrienol, tocopherol, α-tocopherol, tocopherol acetate, tocopheryl linoleic acid or tocopheryl nicotinate
   4) A retinol selected from any one of the following: retinol, retinyl acetate or retinyl palmitate
   5) A menaquinone selected from any one of the following: phylloquinone, menaquinone or menadione
   6) A carotenoid selected from any one of the following: astaxanthin, lutein, fucoxanthin or lycopene
   7) Propolis or a cinnamic acid derivative selected from any one of the following: caffeic acid phenethyl ester, artepillin C or coumaric acid
   8) A diterpene selected from any one of the following: phytol, abietic acid, levopimaric acid or gibberellin
   9) A triterpene selected from any one of the following: squalene, lanosterol, cucurbitacin, limonin or loquat leaf culture extract
   10) Coenzyme Q10
   11) α-lipoic acid
(4) An aqueous solution containing a fat-soluble substance which is produced using one of the methods specified in (1) - (3) above.
(5) An aqueous solution specified in (4) above which is characterized by increased absorbance of the fat-soluble substance by the skin.
(6) An aqueous solution specified in either (4) or (5) above which contains 9 µg / mL or greater curcumin.
(7) An aqueous solution specified in either (4) or (5) above which contains 5 µg / mL or greater coenzyme Q10.
(8) An aqueous solution specified in either (4) or (5) above which contains 0.8 mg / mL or greater α-lipoic acid.
(9) An aqueous solution specified in either (4) or (5) above which contains 5.0 µg / mL or greater retinol.
(10) An aqueous solution specified in either (4) or (5) above which contains 1.0 mg / mL or greater ferulic acid.
(11) An aqueous solution specified in either (4) or (5) above which contains 0.01% (w/v) or greater isoflavone.
(12) An aqueous solution specified in either (4) or (5) above which contains 0.1% (w/w) or greater rose polyphenol.
(13) An aqueous solution specified in either (4) or (5) above which contains 150 µg / mL or greater caffeic acid phenethyl ester.
(14) An aqueous solution specified in either (4) or (5) above which contains 0.05% (w/w) or greater propolis.
(15) An aqueous solution specified in either (4) or (5) above which contains 0.01% (w/w) or greater loquat leaf culture extract.
(16) An aqueous solution specified in either (4) or (5) above which contains 19 mg / mL or greater nobiletin.
(17) An aqueous solution specified in either (4) or (5) above which contains 3.3 µg / mL or greater α-tocopherol and / or 4.5 µg / mL or greater γ-tocotrienol.
(18) A cosmetic manufactured using an aqueous solution specified in (4) - (17) above as an ingredient.
(19) A food product manufactured using an aqueous solution specified in (4) - (17) above as an ingredient.
(20) A pharmaceutical product manufactured using an aqueous solution specified in (4) - (17) above as an ingredient.

### Effects of the Invention

The production method constituted by the present invention enables the easy provision of an aqueous solution which allows for the incorporation of fat-soluble substances useful in human applications, such as curcumin, CoQ10 and α-lipoic acid at high concentrations. Furthermore, the aqueous solution obtained via the present invention is characterized in that absorbance of the fat-soluble substance through the skin is high, and it can be used as a useful ingredient in cosmetics, food products and pharmaceutical products.

### Mode for Implementing the Invention

The "method for producing an aqueous solution containing a fat-soluble substance" constituted by the present invention refers to a method for manufacturing an "aqueous solution containing a fat-soluble substance" in which a fat-soluble substance which is not soluble or barely soluble in water exists in a disperse state in water without manifesting any aggregation or precipitation.
Here, water which is used as a solvent includes pure water, ultrapure water and ion-exchanged water. Water which is used as a solvent in the present invention can be appropriately selected based on the intended use, such as in cosmetics, food products or pharmaceutical products.
The "method for producing an aqueous solution containing a fat-soluble substance" constituted by the present invention should be a production method in which an "aqueous solution containing a fat-soluble substance in high concentration" is obtained via a process in which a super solubilizer, CD and a fat-soluble substance are combined and added to water. The method constituted by the present invention may furthermore include previously known methods for the purposes of producing an "aqueous solution containing a fat-soluble substance".
The "method for producing an aqueous solution containing a fat-soluble substance" constituted by the present invention makes it possible to obtain an "aqueous solution containing a fat-soluble substance at high concentration" by either combining a super solubilizer, cyclodextrin and a fat-soluble substance in water or combining a super solubilizer with a cyclodextrin complex containing a fat-soluble substance in water. Using the method constituted by the present invention, it is possible to produce an aqueous solution which is capable of containing a fat-soluble substance at high concentration.
For the "method for the production of an aqueous solution containing a fat-soluble substance" constituted by the present invention, when combining the fat-soluble substance with CD, the two components my be simply mixed together or kneaded together.
The entirety of the fat-soluble substance and CD may be of the form of a complex of fat-soluble substance and CD formed via the aforementioned mixing or kneading processes. Furthermore, the fat-soluble substance and only some of the CD used may be in complex form.

The "method for the production of an aqueous solution containing a fat-soluble substance" constituted by the present invention may be a method which allows for the ultimate production of the "aqueous solution containing a fat-soluble substance" constituted by the present invention. The "method for the production of an aqueous solution containing a fat-soluble substance" constituted by the current invention may also include the production of an "aqueous solution containing a fat-soluble substance" constituted by the current invention in which a composition containing a super solubilizer is combined with a composition containing a fat-soluble substance and CD which are mixed together at the time of use.
For example, the present invention includes instances in which a cosmetic toner, cream or lotion is prepared from a composition which includes a super solubilizer and, after this composition is applied to the skin, a cosmetic toner, cream or lotion prepared from a composition which includes both a fat-soluble substance and CD is further applied on top of the previous composition, to produce an aqueous solution containing a fat-soluble substance on top of the user's skin. The "aqueous solution containing a fat-soluble substance" thus produced can be directly used as a cosmetic product, etc.

The "super solubilizer" constituted by the present invention refers to an agent which aids in the dissolution of the "fat-soluble substance" such that the fat-soluble substance, which is not soluble or barely soluble in water, can exist in a disperse state in water without manifesting any aggregation or precipitation. The "super solubilizer" constituted by the present invention is able to increase the solubility of the fat-soluble substance in water once combined with CD.
The "super solubilizer" constituted by the previous invention may include any conventionally known agent as long as the agent is useful in a "method for the production of an aqueous solution containing a fat-soluble substance".
Examples of such a "super solubilizer" include, for example 1) an alkali salt or bile acid selected from any one of the following: taurocholate, glycocholate, cholic acid, lithocholic acid or deoxycholic acid, 2) glycosides of polyphenols or hesperidin, 3) an emulsifier selected from any one of the following: glycerin fatty acid ester, sorbitan fatty acid ester, propylene glycol fatty acid ester, or sucrose fatty acid ester, 4) a lecithin agent selected from any one of the following: enzymatically decomposed lecithin, lecithin derived from soybean or lecithin derived from egg yolk, 5) a saponin selected from any one of the following: glycyrrhizic acid or an alkali salt thereof, soybean saponin or quillaja saponin, 6) a casein or alkali salt thereof, 7) a polysorbate (fatty acid polyoxyethylene sorbitan), 8) a cellulose or alkali salt thereof selected from any one of the following: methylcellulose, carboxymethyl cellulose or hydroxyethyl cellulose, or 9) a polysaccharide thickener selected from any one of the following: pectin, carrogeenan or xanthan gum.
The "aqueous solution containing a fat-soluble substance" constituted by the present invention may include one or two or more of these super solubilizers.

The "fat-soluble substance" constituted by the present invention may be any fat-soluble substance which cannot be dissolved or cannot be easily dissolved in water, and should preferably be able to form a complex with CD. Examples of the "fat-soluble substance" constituted by the present invention include polyphenols, flavanoids, tocopherols, retinols, menaquinones, carotenoids, cinnamic acid derivatives or propolis, diterpenes, terpenes, CoQ10 and α-lipoic acid.

Specifically, examples of the "fat-soluble substance" constituted by the present invention include 1) a polyphenol selected from any one of the following:
curcumin, rose polyphenol,
Ferulic acid, quercetin or cacao mass; 2) a flavanoid selected from any one of the following: isoflavone, isoflavone aglycone, flavanone, anthocyanidin, catechin, chalcone or nobiletin; 3) a tocopherol selected from any one of the following: tocotrienol, γ-tocotrienol, tocopherol, α-tocopherol, tocopherol acetate, tocopheryl linoleic acid or tocopheryl nicotinate; 4) a retinol selected from any one of the following: retinol, retinyl acetate or retinyl palmitate; 5) a menaquinone selected from any one of the following: phylloquinone, menaquinone or menadione; 6) a carotenoid selected from any one of the following: astaxanthin, lutein, fucoxanthin or lycopene; 7) propolis or a cinnamic acid derivative selected from any one of the following: CAPE, artepillin C or coumaric acid; 8) a diterpene selected from any one of the following: phytol, abietic acid, levopimaric acid or gibberellin; 9) a triterpene selected from any one of the following: squalene, lanosterol, cucurbitacin, limonin or loquat leaf culture extract; 10) CoQ10; and 11) α-lipoic acid. The "aqueous solution containing a fat-soluble substance" constituted by the present invention may include one or two or more of these "fat-soluble substances".

For the present invention, the CD which is combined with one of these "fat-soluble substances" or which is formed into a "cyclodextrin complex containing a fat-soluble substance" may be any conventionally known CD as long as the CD is useful for the production of the "aqueous solution containing a fat-soluble substance" constituted by the present invention.
Examples of this CD include αCD, βCD, γCD and branched CD.
Examples of branched CD include, for example, maltosyl-α-CD (hereafter sometimes referred to as "M-αCD"), maltosyl-β-CD (hereafter sometimes referred to as "M-βCD"), maltosyl-γ-CD (hereafter sometimes referred to as "M-γCD"), glucosyl-α-CD, glucosyl-β-CD and glucosyl-γ-CD. Furthermore it is possible to use a mixture of the aforementioned substances as a branched CD.
The "aqueous solution containing a fat-soluble substance" constituted by the present invention may be a substance that combines one or two or more of the aforementioned "fat-soluble substances" with one or two or more of these "CDs".
In the "method for the production of an aqueous solution containing a fat-soluble substance" constituted by the present invention, physical treatment, such as stirring, treatment via heating or treatment via mixing may be used as necessary depending on the type of fat-soluble substance which is dissolved, in order to ensure that the fat-soluble substance can exist in a disperse state in water. Mixing and stirring devices which may be used include commonly used devices such as a shaking agitator, ultrasonic device, magnetic stirrer, mechanical stirrer, vortex mixer, Ultra-Turrax ^{®} or high-pressure homogenizer.

The temperature at which the "method for the production of an aqueous solution containing a fat-soluble substance" constituted by the present invention is carried out, is not restricted to any particular temperature provided the temperature allows for the production of the aqueous solution containing a fat-soluble substance constituted by the present invention. This implementation temperature should, for example, preferably range from 10 - 80°C with a range of 15 - 40°C more preferable and room temperature (approximately 25°C) even more preferable.
The stirring time is not restricted to any specific period of time, as long as the stirring time used allows for the production of the aqueous solution containing a fat-soluble substance constituted by the present invention, but stirring may be carried out over a short period of time. If the "aqueous solution containing a fat-soluble substance" being produced is small in quantity, a stirring time of approximately 5 seconds is sufficient, and larger quantities should preferably be stirred for less than 24 hours. Stirring time should preferably range from 1 to 8 hours and a 1 - 6 hour range is even more preferable.

The "aqueous solution containing a fat-soluble substance" thus obtained from the "method for the production of an aqueous solution containing a fat-soluble substance" constituted by the present invention may be an aqueous solution which is capable of containing a fat-soluble substance at high concentration due to increased solubility of the fat-soluble substance in water. Furthermore, it may be an aqueous solution which is characterized in that "absorbance of the fat-soluble substance by the skin is increased".
Here, "increased absorbance of the fat-soluble substance by the skin" refers to a state in which the amount of fat-soluble substance absorbed by the skin when the aqueous solution constituted by the present invention is applied to the skin, including the epidermis and dermis, of a mammal, including humans, is several times, 4 times, 20 times or 40 times, etc. greater than preparations in which a fat-soluble substance has simply been dissolved or suspended in water. The aqueous solution for which "absorbance of the fat-soluble substance by the skin is increased" which is constituted by the present invention has the advantage of being able to efficiently incorporate useful fat-soluble substances into the skin, including the epidermis and dermis.

Among the "aqueous solutions containing a fat-soluble substance" obtained via the "method for the production of an aqueous solution containing a fat-soluble substance" constituted by the present invention, examples of "aqueous solutions containing curcumin" include aqueous solutions containing, for example, 9 µg / mL or greater, 12 µg / mL or greater, or 15 µg / mL or greater curcumin. Furthermore, examples of "an aqueous solution containing curcumin" include aqueous solutions which contain 50 µg / mL or greater or 100 µg / mL or greater curcumin. Furthermore, an aqueous solution containing a high concentration of curcumin (150 µg / mL or greater) may also be used.
Upon obtaining such an aqueous solution containing curcumin constituting the present invention, the solution may be combined with any of the super solubilizers referred to in the present invention. For example, sodium taurocholate (hereafter sometimes referred to as NaTC), glycyrrhizic acid (hereafter sometimes referred to as GZ), soybean saponin (hereafter sometimes referred to as SS), quillaja saponin (hereafter sometimes referred to as QS), polysorbate, methylcellulose (hereafter sometimes referred to as MC), glycyrrhizin K₂ (hereafter sometimes referred to as GZK₂) or sodium glycocholate (hereafter sometimes referred to as Na glycocholate) may be combined with the "method for the production of an aqueous solution containing a fat-soluble substance" constituted by the present invention.
Examples of polysorbates include, for example, commercially available polysorbate 20 or polysorbate 80. Examples of methylcellulose include, for example, commercially available methylcellulose 15, methylcellulose 50, methylcellulose 100 and methylcellulose 400. This polysorbate or methylcellulose is prepared as an aqueous solution and can be combined to create the super solubilizer constituting the present invention.

In the event that, upon production of the "aqueous solution containing curcumin" constituting the present invention, the super solubilizer which is combined is NaTC, NaTC can be added such that it comprises 0.1% (w/v) or greater of the total amount of "aqueous solution containing curcumin" which is produced. Because the "method for the production of an aqueous solution containing a fat-soluble substance" constituted by the present invention increases the solubility of curcumin in a concentration-dependent manner based on the amount of NaTC added, 0.5% (w/v) or greater or 1.0% (w/v) or greater NaTC can be added.
By incorporating NaTC as a super solubilizer, an aqueous solution containing 9 µg / mL or greater, 12 µg / mL or greater or 15 µg / mL or greater curcumin, an aqueous solution containing 50 µg / mL or greater or 100 µg / mL or greater curcumin, or, furthermore, an aqueous solution containing a high concentration of curcumin (150 µg / mL or greater) can be produced.

In the event that, upon production of the "aqueous solution containing curcumin" constituting the present invention, the super solubilizer which is combined is GZ, GZ can be added such that it comprises 1.0% (w/v) or greater of the total amount of "aqueous solution containing curcumin" which is produced. Because the "method for the production of an aqueous solution containing a fat-soluble substance" constituted by the present invention increases the solubility of curcumin in a concentration-dependent manner based on the amount of GZ added, 2.0% (w/v) or greater or 3.0% (w/v) or greater GZ can be added.
By incorporating GZ as a super solubilizer, an aqueous solution containing 9 µg / mL or greater, 12 µg / mL or greater or 15 µg / mL or greater curcumin, an aqueous solution containing 50 µg / mL or greater or 100 µg / mL or greater curcumin, or, furthermore, an aqueous solution containing a high concentration of curcumin (150 µg / mL or greater) can be produced.
Note, however, that since GZ causes gelation at high concentrations, the amount of GZ added should preferably not exceed 10% (w/v).

In the event that, upon production of the "aqueous solution containing curcumin" constituting the present invention, the super solubilizer which is combined is SS, SS can be added such that it comprises 0.1% (w/v) or greater of the total amount of "aqueous solution containing curcumin" which is produced. Because the "method for the production of an aqueous solution containing a fat-soluble substance" constituted by the present invention increases the solubility of curcumin in a concentration-dependent manner based on the amount of SS added, 0.5% (w/v) or greater or 1.0% (w/v) or greater SS can be added.
By incorporating SS as a super solubilizer, an aqueous solution containing 9 µg / mL or greater, 12 µg / mL or greater or 15 µg / mL or greater curcumin, an aqueous solution containing 50 µg / mL or greater or 100 µg / mL or greater curcumin, or, furthermore, an aqueous solution containing a high concentration of curcumin (150 µg / mL or greater) can be produced.

In the event that, upon production of the "aqueous solution containing curcumin" constituting the present invention, the super solubilizer which is combined is QS, QS can be added such that it comprises 0.5% (v/v) or greater of the total amount of "aqueous solution containing curcumin" which is produced. Because the "method for the production of an aqueous solution containing a fat-soluble substance" constituted by the present invention increases the solubility of curcumin in a concentration-dependent manner based on the amount of QS added, 2.5% (v/v) or greater or 5.0% (v/v) or greater QS can be added.
By incorporating QS as a super solubilizer, an aqueous solution containing 9 µg / mL or greater, 12 µg / mL or greater or 15 µg / mL or greater curcumin, an aqueous solution containing 50 µg / mL or greater or 100 µg / mL or greater curcumin, or, furthermore, an aqueous solution containing a high concentration of curcumin (150 µg / mL or greater) can be produced.

In the event that, upon production of the "aqueous solution containing curcumin" constituting the present invention, the super solubilizer which is combined is polysorbate 80, an aqueous solution of polysorbate 80 (hereafter sometimes referred to as polysorbate 80 aqueous solution)
can be added such that the concentration of polysorbate 80 is 0.1% (w/v) or greater of the total amount of "aqueous solution containing curcumin" which is produced. Because the "method for the production of an aqueous solution containing a fat-soluble substance" constituted by the present invention increases the solubility of curcumin in a concentration-dependent manner based on the amount of polysorbate 80 added, polysorbate 80 aqueous solution can be added such that the concentration of polysorbate 80 is 0.5% (w/v) or greater or 1.0% (w/v) or greater.
Furthermore, when adding an aqueous solution of polysorbate 20 (hereafter sometimes referred to as polysorbate 20 aqueous solution), the polysorbate 20 may be added such that the concentration of polysorbate 20 is 0.1% (w/v) or greater of the total amount of "aqueous solution containing curcumin" which is produced. Because the "method for the production of an aqueous solution containing a fat-soluble substance" constituted by the present invention increases the solubility of curcumin in a concentration-dependent manner based on the amount of polysorbate 20 added, polysorbate 20 aqueous solution can be added such that the concentration of polysorbate 20 is 0.5% (w/v) or greater or 1.0% (w/v) or greater. Furthermore, using the "method for the production of an aqueous solution containing a fat-soluble substance" constituted by the present invention, it is possible to combine both polysorbate 80 and polysorbate 20 for addition as a super solubilizer.
By incorporating polysorbate as a super solubilizer, an aqueous solution containing 9 µg / mL or greater, 12 µg / mL or greater or 15 µg / mL or greater curcumin, an aqueous solution containing 50 µg / mL or greater or 100 µg / mL or greater curcumin, or, furthermore, an aqueous solution containing a high concentration of curcumin (150 µg / mL or greater) can be produced.
Note, however, that the use of a high concentration of polysorbate will cause the viscosity of the "aqueous solution containing curcumin" to increase, making use difficult, so the amount of polysorbate added should preferably be less than 5.0% (w/v) of the total amount of "aqueous solution containing curcumin" which is produced.

In the event that, upon production of the "aqueous solution containing curcumin" constituting the present invention, the super solubilizer which is combined is methylcellulose, an aqueous solution of methylcellulose can be added such that it comprises 0.5% (w/v) or greater of the total amount of "aqueous solution containing curcumin" which is produced.
Examples of aqueous solutions of methylcellulose include aqueous solutions of methylcellulose 15 (hereafter sometimes referred to as methylcellulose 15 aqueous solution), aqueous solutions of methylcellulose 50 (hereafter sometimes referred to as methylcellulose 50 aqueous solution), aqueous solutions of methylcellulose 100 (hereafter sometimes referred to as methylcellulose 100 aqueous solution), and aqueous solutions of methylcellulose 400 (hereafter sometimes referred to as methylcellulose 400 aqueous solution).
Because the "method for the production of an aqueous solution containing a fat-soluble substance" constituted by the present invention increases the solubility of curcumin in a concentration-dependent manner based on the amount of methylcellulose aqueous solution added, methylcellulose aqueous solution can be added such that the concentration of methylcellulose is 1.0% (w/v) or greater or 2.0% (w/v) or greater. Furthermore, the "method for the production of an aqueous solution containing a fat-soluble substance" constituting the present invention allows for the combination of different varieties of methylcellulose, such as methylcellulose 15 aqueous solution and methylcellulose 50 aqueous solution, for concurrent addition as a super solubilizer.
By incorporating methylcellulose as a super solubilizer, an aqueous solution containing 9 µg / mL or greater, 12 µg / mL or greater or 15 µg / mL or greater curcumin, an aqueous solution containing 50 µg / mL or greater or 100 µg / mL or greater curcumin, or, furthermore, an aqueous solution containing a high concentration of curcumin (150 µg / mL or greater) can be produced.
Note, however, that viscosity increases if methylcellulose is present in high concentrations, making use difficult, so the amount of methylcellulose added should preferably not exceed 5.0% (w/v).

In the event that, upon production of the "aqueous solution containing curcumin" constituting the present invention, the super solubilizer which is combined is GZK₂, GZK₂ can be added such that it comprises 1.0% (w/v) or greater of the total amount of "aqueous solution containing curcumin" which is produced. Because the "method for the production of an aqueous solution containing a fat-soluble substance" constituted by the present invention increases the solubility of curcumin in a concentration-dependent manner based on the amount of GZK₂ added, 2.0% (w/v) or greater or 3.0% (w/v) or greater GZK₂ can be added.
By incorporating GZK₂ as a super solubilizer, an aqueous solution containing 9 µg / mL or greater, 12 µg / mL or greater or 15 µg / mL or greater curcumin, an aqueous solution containing 50 µg / mL or greater or 100 µg / mL or greater curcumin, or, furthermore, an aqueous solution containing a high concentration of curcumin (150 µg / mL or greater) can be produced.
Note, however, that since GZK₂ causes gelation at high concentrations, the amount of GZK₂ added should preferably not exceed 10% (w/v).

In the event that, upon production of the "aqueous solution containing curcumin" constituting the present invention, the super solubilizer which is combined is Na glycocholate, Na glycocholate can be added such that it comprises 0.1% (w/v) or greater of the total amount of "aqueous solution containing curcumin" which is produced. Because the "method for the production of an aqueous solution containing a fat-soluble substance" constituted by the present invention increases the solubility of curcumin in a concentration-dependent manner based on the amount of Na glycocholate added, 0.5% (w/v) or greater or 1.0% (w/v) or greater Na glycocholate can be added.
By incorporating Na glycocholate as a super solubilizer, an aqueous solution containing 9 µg / mL or greater, 12 µg / mL or greater or 15 µg / mL or greater curcumin, an aqueous solution containing 50 µg / mL or greater or 100 µg / mL or greater curcumin, or, furthermore, an aqueous solution containing a high concentration of curcumin (150 µg / mL or greater) can be produced.

CD which is combined with the super solubilizer constituted by the present invention may be any conventionally known substance as long as it is useful in the production of the "aqueous solution containing curcumin" which is constituted by the present invention, but γCD is preferable. In particular, a "curcumin-γCD complex" which includes curcumin in γCD is preferable and any conventionally known substance may be used as this "curcumin-γCD complex". For example, commercially available CAVAMAX^{®} W8 Curcumin (Wacker Chemical Ltd.) represents one type of CD which can be combined with the super solubilizer characterized by the present invention.

Among the "aqueous solutions containing a fat-soluble substance" obtained via the "method for the production of an aqueous solution containing a fat-soluble substance" constituted by the present invention, examples of "aqueous solutions containing CoQ10" include aqueous solutions containing, for example, 5 µg / mL or greater, 10 µg / mL or greater, or 20 µg / mL or greater CoQ10.
Upon obtaining such an aqueous solution containing CoQ10 constituting the present invention, the solution may be combined with any of the super solubilizers referred to in the present invention. For example, NaTC or GZK₂ may be combined with the "method for the production of an aqueous solution containing a fat-soluble substance" constituted by the present invention.

In the event that, upon production of the "aqueous solution containing CoQ10" constituting the present invention, the super solubilizer which is combined is NaTC, NaTC can be added such that it comprises 0.1% (w/v) or greater of the total amount of "aqueous solution containing CoQ10" which is produced. Because the "method for the production of an aqueous solution containing a fat-soluble substance" constituted by the present invention increases the solubility of CoQ10 in a concentration-dependent manner based on the amount of NaTC added, 0.5% (w/v) or greater or 1.0% (w/v) or greater NaTC can be added.
By combining NaTC as a super solubilizer, it is possible to produce an aqueous solution containing 5 µg / mL or greater, 10 µg / mL or greater, or 20 µg / mL or greater CoQ10.

In the event that, upon production of the "aqueous solution containing CoQ10" constituting the present invention, the super solubilizer which is combined is GZK₂, GZK₂ can be added such that it comprises 0.03% (w/w) or greater of the total amount of "aqueous solution containing CoQ10" which is produced. Because the "method for the production of an aqueous solution containing a fat-soluble substance" constituted by the present invention increases the solubility of CoQ10 in a concentration-dependent manner based on the amount of GZK₂ added, 0.16% (w/w) or greater or 0.33% (w/w) or greater GZK₂ can be added.
By combining GZK₂ as a super solubilizer, it is possible to produce an aqueous solution containing 5 µg / mL or greater, 10 µg / mL or greater, or 20 µg / mL or greater CoQ10.
Note, however, that since GZK₂ causes gelation at high concentrations, the amount of GZK₂ added should preferably not exceed 10% (w/v).

CD which is combined with the super solubilizer constituted by the present invention may be any conventionally known substance as long as it is useful in the production of the "aqueous solution containing CoQ10" which is constituted by the present invention, but γCD is preferable. In particular, a "CoQ10-γCD complex" which includes CoQ10 in γCD is preferable and any conventionally known substance may be used as this "curcumin-CoQ10 complex". For example, commercially available CAVAMAX^{®} W8 CoQ10 (Wacker Chemical Ltd.) represents one type of CD which can be combined with the super solubilizer characterized by the present invention.

Among the "aqueous solutions containing a fat-soluble substance" obtained via the "method for the production of an aqueous solution containing a fat-soluble substance" constituted by the present invention, examples of "aqueous solutions containing α-lipoic acid" include aqueous solutions containing, for example, 0.8 mg / mL or greater, 1.0 mg / mL or greater, or 1.2 mg / mL or greater α-lipoic acid.
When using α-lipoic acid, the α-lipoic acid will dissolve in water, but it will not dissolve completely and will partially exist as an aggregate. However, by combining α-lipoic acid with super solubilizer and CD in the present invention, it is possible to obtain an aqueous solution into which α-lipoic acid is dissolved at a concentration of 0.8 mg / mL without forming aggregates or precipitates.
Upon obtaining such an aqueous solution containing α-lipoic acid which does not form aggregates, the solution may be combined with any of the super solubilizers referred to in the present invention. For example, NaTC, etc. may be combined with the "method for the production of an aqueous solution containing a fat-soluble substance" constituted by the present invention.

In the event that, upon production of the "aqueous solution containing α-lipoic acid" constituting the present invention, the super solubilizer which is combined is NaTC, NaTC can be added such that it comprises 0.5% (w/v) or greater of the total amount of "aqueous solution containing α-lipoic acid" which is produced. Because the "method for the production of an aqueous solution containing a fat-soluble substance" constituted by the present invention increases the solubility of α-lipoic acid in a concentration-dependent manner based on the amount of NaTC added, 1.0% (w/v) or greater or 2.0% (w/v) or greater NaTC can be added.
By combining NaTC as a super solubilizer, it is possible to produce an aqueous solution containing 0.8 mg / mL or greater, 1.0 mg / mL or greater, or 1.2 mg / mL or greater α-lipoic acid.

CD which is combined with the super solubilizer constituted by the present invention may be any conventionally known substance as long as it is useful in the production of the "aqueous solution containing α-lipoic acid" which is constituted by the present invention, but γCD is preferable. In particular, an "α-lipoic acid-γCD complex" which includes α-lipoic acid in γCD is preferable and any conventionally known substance may be used as this "α-lipoic acid-γCD complex". For example, commercially available α-lipoic acid CD-20 (Mitsubishi-Kagaku Foods) or R-α-lipoic acid CD (CycloChem Bio), can be used as CDs which are combined with the super solubilizer constitued by the present invention.

Among the "aqueous solutions containing a fat-soluble substance" obtained via the "method for the production of an aqueous solution containing a fat-soluble substance" constituted by the present invention, examples of "aqueous solutions containing retinol" include aqueous solutions containing, for example, 5 µg / mL or greater, 10 µg / mL or greater, or 20 µg / mL or greater retinol.
Upon obtaining such an "aqueous solution containing retinol" constituting the present invention, the solution may be combined with any of the super solubilizers referred to in the present invention. For example, GZK₂, etc. may be combined with the "method for the production of an aqueous solution containing a fat-soluble substance" constituted by the present invention.

In the event that, upon production of the "aqueous solution containing retinol" constituting the present invention, the super solubilizer which is combined is GZK₂, GZK₂ can be added such that it comprises 0.03% (w/w) or greater of the total amount of "aqueous solution containing retinol" which is produced. Because the "method for the production of an aqueous solution containing a fat-soluble substance" constituted by the present invention increases the solubility of retinol in a concentration-dependent manner based on the amount of GZK₂ added, 0.16% (w/w) or greater or 0.33% (w/w) or greater GZK₂ can be added.
By combining GZK₂ as a super solubilizer, it is possible to produce an aqueous solution containing 5 µg / mL or greater, 10 µg / mL or greater, or 20 µg / mL or greater retinol.
Note, however, that since GZK₂ causes gelation at high concentrations, the amount of GZK₂ added should preferably not exceed 10% (w/v).

CD which is combined with the super solubilizer constituted by the present invention may be any conventionally known substance as long as it is useful in the production of the aqueous solution containing retinol which is constituted by the present invention, but γCD is preferable. In particular, a "retinol-γCD complex" which includes retinol in γCD is preferable and any conventionally known substance may be used as this "retinol-γCD complex". For example, commercially available CAVAMAX^{®} W8 Retinol (Wacker Chemical Ltd.) represents one type of CD which can be combined with the super solubilizer characterized by the present invention.

Among the "aqueous solutions containing a fat-soluble substance" obtained via the "method for the production of an aqueous solution containing a fat-soluble substance" constituted by the present invention, examples of "aqueous solutions containing ferulic acid" include aqueous solutions containing, for example, 1.0 mg / mL or greater, 5.0 mg / mL or greater, or 10 mg / mL or greater ferulic acid.
Upon obtaining such an "aqueous solution containing ferulic acid" constituting the present invention, the solution may be combined with any of the super solubilizers referred to in the present invention. For example, NaTC, etc. may be combined with the "method for the production of an aqueous solution containing a fat-soluble substance" constituted by the present invention.

In the event that, upon production of the "aqueous solution containing ferulic acid" constituting the present invention, the super solubilizer which is combined is NaTC, NaTC can be added such that it comprises 0.5% (w/v) or greater of the total amount of "aqueous solution containing ferulic acid" which is produced. Because the "method for the production of an aqueous solution containing a fat-soluble substance" constituted by the present invention increases the solubility of ferulic acid in a concentration-dependent manner based on the amount of NaTC added, 1.0% (w/v) or greater or 2.0% (w/v) or greater NaTC can be added.
By combining NaTC as a super solubilizer, it is possible to produce an aqueous solution containing 1.0 mg / mL or greater, 5.0 mg / mL or greater, or 10 mg / mL or greater ferulic acid.

CD which is combined with the super solubilizer constituted by the present invention may be any conventionally known substance as long as it is useful in the production of the "aqueous solution containing ferulic acid" which is constituted by the present invention. Examples of the above include M-αCD, αCD and γCD and for these commercially available products such as M-αCD (Fuji Nihon Seito), αCD (CAVAMAX® W6 Food) and γCD (CAVAMAX® W8 Food) may be used. Ferulic acid may be any conventionally known substance, and commercially available ferulic acid (Tsuno Co., Ltd.), for example, may be incorporated with the "method for the production of an aqueous solution containing a fat-soluble substance" constituted by the present invention.

Among the "aqueous solutions containing a fat-soluble substance" obtained via the "method for the production of an aqueous solution containing a fat-soluble substance" constituted by the present invention, examples of "aqueous solutions containing isoflavone" include aqueous solutions containing, for example, 0.01% (w/v) or greater, 0.02% (w/v) or greater or 0.03% (w/v) or greater isoflavone.
Upon obtaining such an "aqueous solution containing isoflavone" constituting the present invention, the solution may be combined with any of the super solubilizers referred to in the present invention. For example, methylcellulose such as methylcellulose 400, SS or GZ may be combined with the "method for the production of an aqueous solution containing a fat-soluble substance" constituted by the present invention. Methylcellulose may also be combined as an aqueous solution of methylcellulose 400.

In the event that, upon production of the "aqueous solution containing isoflavone" constituting the present invention, the super solubilizer which is combined is methylcellulose, an aqueous solution of methylcellulose can be added such that it comprises 0.19% (w/w) or greater of the total amount of "aqueous solution containing isoflavone" which is produced. Examples of aqueous solutions of methylcellulose include methylcellulose 400, etc.
Because the "method for the production of an aqueous solution containing a fat-soluble substance" constituted by the present invention increases the solubility of isoflavone in a concentration-dependent manner based on the amount of methylcellulose aqueous solution added, methylcellulose aqueous solution can be added such that the concentration of methylcellulose is 0.39% (w/w) or greater or 0.98% (w/w) or greater.
By combining methylcellulose as a super solubilizer, it is possible to produce an aqueous solution containing 0.01% (w/v) or greater, 0.02% (w/v) or greater, or 0.03% (w/v) or greater isoflavone.
Note, however, that viscosity increases if methylcellulose is present in high concentrations, making use difficult, so the amount of methylcellulose added should preferably not exceed 5.0% (w/w).

In the event that, upon production of the "aqueous solution containing isoflavone" constituting the present invention, the super solubilizer which is combined is SS, SS can be added such that it comprises 0.2% (w/w) or greater of the total amount of "aqueous solution containing isoflavone" which is produced. Because the "method for the production of an aqueous solution containing a fat-soluble substance" constituted by the present invention increases the solubility of isoflavone in a concentration-dependent manner based on the amount of SS added, 0.3% (w/w) or greater or 0.4% (w/w) or greater SS can be added.
By combining SS as a super solubilizer, it is possible to produce an aqueous solution containing 0.01% (w/v) or greater, 0.02% (w/v) or greater, or 0.03% (w/v) or greater isoflavone.

In the event that, upon production of the "aqueous solution containing isoflavone" constituting the present invention, the super solubilizer which is combined is GZ, GZ can be added such that it comprises 0.2% (w/w) or greater of the total amount of "aqueous solution containing isoflavone" which is produced. Because the "method for the production of an aqueous solution containing a fat-soluble substance" constituted by the present invention increases the solubility of isoflavone in a concentration-dependent manner based on the amount of GZ added, 0.3% (w/w) or greater or 0.4% (w/w) or greater GZ can be added.
By combining GZ as a super solubilizer, it is possible to produce an aqueous solution containing 0.01% (w/v) or greater, 0.02% (w/v) or greater, or 0.03% (w/v) or greater isoflavone.
Note, however, that since GZ causes gelation at high concentrations, the amount of GZ added should preferably not exceed 10% (w/v).

CD which is combined with the super solubilizer constituted by the present invention may be any conventionally known substance as long as it is useful in the production of the "aqueous solution containing isoflavone" which is constituted by the present invention, but γCD is preferable. In particular, an "isoflavone-γCD complex" which includes isoflavone in γCD is preferable and any substance prepared from isoflavone such as the commercially available AglyMax^{®}-30 (total isoflavone aglycone 34.7%, Nichimo Biotics) and γCD such as CAVAMAX® W8 Food may be used as this "isoflavone-γCD complex."

Among the "aqueous solutions containing a fat-soluble substance" obtained via the "method for the production of an aqueous solution containing a fat-soluble substance" constituted by the present invention, examples of "aqueous solutions containing rose polyphenol, include aqueous solutions containing, for example, 0.1% (w/w) or greater, 0.2% (w/w) or greater or 0.3% (w/w) or greater rose polyphenol.
Upon obtaining such an "aqueous solution containing rose polyphenol" constituting the present invention, the solution may be combined with any of the super solubilizers referred to in the present invention. For example, NaTC, etc. may be combined with the "method for the production of an aqueous solution containing a fat-soluble substance" constituted by the present invention.

In the event that, upon production of the "aqueous solution containing rose polyphenol" constituting the present invention, the super solubilizer which is combined is NaTC, NaTC can be added such that it comprises 0.01% (w/v) or greater of the total amount of "aqueous solution containing rose polyphenol" which is produced. Because the "method for the production of an aqueous solution containing a fat-soluble substance" constituted by the present invention increases the solubility of rose polyphenol in a concentration-dependent manner based on the amount of NaTC added, 0.05% (w/v) or greater or 0.1% (w/v) or greater NaTC can be added.
By combining NaTC as a super solubilizer, it is possible to produce an aqueous solution containing 0.1% (w/w) or greater, 0.2% (w/w) or greater, or 0.3% (w/w) or greater rose polyphenol.

CD which is combined with the super solubilizer constituted by the present invention may be any conventionally known substance as long as it is useful in the production of the "aqueous solution containing rose polyphenol" which is constituted by the present invention, but αCD is preferable. For αCD, any conventionally known substance may be used, including, for example commercially available products such as CAVAMAX^{®} W6 Food. For rose polyphenol, as well, any conventionally known substance may be used, including, for example the commercially available rose polyphenol product ROSE CRYSTA^{®}-70 (70% or greater rose polyphenol content, ToyoHakko Co., Ltd.).

Among the "aqueous solutions containing a fat-soluble substance" obtained via the "method for the production of an aqueous solution containing a fat-soluble substance" constituted by the present invention, examples of "aqueous solutions containing CAPE" include aqueous solutions containing, for example, 150 µg / mL or greater, 170 µg / mL or greater, or 200 µg / mL or greater CAPE.
Upon obtaining such an "aqueous solution containing CAPE" constituting the present invention, the solution may be combined with any of the super solubilizers referred to in the present invention. For example, NaTC, etc. may be combined with the "method for the production of an aqueous solution containing a fat-soluble substance" constituted by the present invention.

In the event that, upon production of the "aqueous solution containing CAPE" constituting the present invention, the super solubilizer which is combined is NaTC, NaTC can be added such that it comprises 0.1% (w/v) or greater of the total amount of "aqueous solution containing CAPE" which is produced. Because the "method for the production of an aqueous solution containing a fat-soluble substance" constituted by the present invention increases the solubility of CAPE in a concentration-dependent manner based on the amount of NaTC added, 0.5% (w/v) or greater or 1.0% (w/v) or greater NaTC can be added.
By combining NaTC as a super solubilizer, it is possible to produce an aqueous solution containing 150 µg / mL or greater, 170 µg / mL or greater, or 200 µg / mL or greater CAPE.

CD which is combined with the super solubilizer constituted by the present invention may be any conventionally known substance as long as it is useful in the production of the "aqueous solution containing CAPE" which is constituted by the present invention, but γCD is preferable. In particular, a "CAPE-γCD complex" which includes CAPE in γCD is preferable and any substance prepared from CAPE such as the commercially available CAPE (Sigma-Aldrich Co., Ltd.) and γCD such as CAVAMAX® W8 Food may be used as this "CAPE-γCD complex".

Among the "aqueous solutions containing a fat-soluble substance" obtained via the "method for the production of an aqueous solution containing a fat-soluble substance" constituted by the present invention, examples of "aqueous solutions containing propolis" include aqueous solutions containing, for example, 0.05% (w/w) or greater, 0.1% (w/w) or greater or 0.15% (w/w) or greater propolis.
Upon obtaining such an "aqueous solution containing propolis" constituting the present invention, the solution may be combined with any of the super solubilizers referred to in the present invention. For example, polysorbate such as polysorbate 80 may be combined with the "method for the production of an aqueous solution containing a fat-soluble substance" constituted by the present invention.

In the event that, upon production of the "aqueous solution containing propolis" constituting the present invention, the super solubilizer which is combined is polysorbate 80, polysorbate 80 aqueous solution can be added such that the concentration of polysorbate 80 is 0.1% (w/v) or greater of the total amount of "aqueous solution containing propolis" which is produced. Because the "method for the production of an aqueous solution containing a fat-soluble substance" constituted by the present invention increases the solubility of propolis in a concentration-dependent manner based on the amount of polysorbate 80 added, polysorbate 80 aqueous solution can be added such that the concentration of polysorbate 80 is 0.2% (w/v) or greater or 0.5% (w/v) or greater.
By combining polysorbate 80 as a super solubilizer, it is possible to produce an aqueous solution containing 0.05% (w/w) or greater, 0.1% (w/w) or greater, or 0.15% (w/w) or greater propolis.
Note, however, that viscosity increases if polysorbate is present in high concentrations, making use difficult, so the amount of polysorbate added should preferably not exceed 5.0% (w/v).

CD which is combined with the super solubilizer constituted by the present invention may be any conventionally known substance as long as it is useful in the production of the "aqueous solution containing propolis" which is constituted by the present invention, but γCD is preferable. In particular, a "propolis-γCD complex" which includes propolis in γCD is preferable and any substance prepared from propolis such as the commercially available Bio30® and γCD such as CAVAMAX® W8 Food may be used as this "propolis-γCD complex".

Among the "aqueous solutions containing a fat-soluble substance" obtained via the "method for the production of an aqueous solution containing a fat-soluble substance" constituted by the present invention, examples of "aqueous solutions containing loquat leaf culture extract" include aqueous solutions containing, for example, 0.01% (w/w) or greater, 0.02% (w/w) or greater or 0.05% (w/w) or greater loquat leaf culture extract.
Upon obtaining such an "aqueous solution containing loquat leaf culture extract" constituting the present invention, the solution may be combined with any of the super solubilizers referred to in the present invention. For example, it is possible to use polysorbates such as polysorbate 80. For polysorbate, polysorbate 80 aqueous solution, etc. may be combined with the "method for the production of an aqueous solution containing a fat-soluble substance" constituted by the present invention.

In the event that, upon production of the "aqueous solution containing loquat leaf culture extract" constituting the present invention, the super solubilizer which is combined is polysorbate 80, polysorbate 80 aqueous solution
can be added such that the concentration of polysorbate 80 is 0.1% (w/v) or greater of the total amount of "aqueous solution containing loquat leaf culture extract" which is produced. Because the "method for the production of an aqueous solution containing a fat-soluble substance" constituted by the present invention increases the solubility of loquat leaf culture extract in a concentration-dependent manner based on the amount of polysorbate 80 added, polysorbate 80 aqueous solution can be added such that the concentration of polysorbate 80 is 0.2% (w/v) or greater or 0.5% (w/v) or greater.
By combining polysorbate 80 as a super solubilizer, it is possible to produce an aqueous solution containing 0.01% (w/w) or greater, 0.02% (w/w) or greater, or 0.05% (w/w) or greater propolis.
Note, however, that viscosity increases if polysorbate is present in high concentrations, making use difficult, so the amount of polysorbate added should preferably not exceed 5.0% (w/v).

CD which is combined with the super solubilizer constituted by the present invention may be any conventionally known substance as long as it is useful in the production of the "aqueous solution containing loquat leaf culture extract" which is constituted by the present invention, but γCD is preferable. In particular, a "loquat leaf culture extract-γCD complex" which includes loquat leaf culture extract in γCD is preferable and any substance prepared from commercially available loquat leaf culture extract and γCD such as CAVAMAX^{®} W8 Food may be used as this "loquat leaf culture extract-γCD complex".

Among the "aqueous solutions containing a fat-soluble substance" obtained via the "method for the production of an aqueous solution containing a fat-soluble substance" constituted by the present invention, examples of "aqueous solutions containing nobiletin" include aqueous solutions containing, for example, 19 mg / mL or greater, 34 mg / mL or greater, 105 mg / mL or greater, or 207 mg / mL or greater nobiletin.
Upon obtaining such an "aqueous solution containing nobiletin" constituting the present invention, the solution may be combined with any of the super solubilizers referred to in the present invention. For example, NaTC, etc. may be combined with the "method for the production of an aqueous solution containing a fat-soluble substance" constituted by the present invention.

In the event that, upon production of this aqueous solution, the super solubilizer which is combined is NaTC, NaTC can be added such that it comprises 0.5% (w/v) or greater of the total amount of "aqueous solution containing nobiletin" which is produced. Because the "method for the production of an aqueous solution containing a fat-soluble substance" constituted by the present invention increases the solubility of nobiletin in a concentration-dependent manner based on the amount of NaTC added, 1.0% (w/v) or greater or 2.0% (w/v) or greater NaTC can be added.
By combining NaTC as a super solubilizer, it is possible to produce an aqueous solution which includes 1.5 times, or, preferably, 2.0 times the amount of nobiletin found in a γCD clathrate for which solubility is increased.

CD which is combined with the super solubilizer constituted by the present invention may be any conventionally known substance as long as it is useful in the production of the "aqueous solution containing nobiletin" which is constituted by the present invention, but γCD is preferable. In particular, a "nobiletin-γCD complex" which includes nobiletin in γCD is preferable and any conventionally known substance may be used as this "nobiletin-γCD complex". For example, commercially available Biletin (Arkray, Inc.) represents one type of CD which can be combined with the super solubilizer characterized by the present invention.

Among the "aqueous solutions containing a fat-soluble substance" obtained via the "method for the production of an aqueous solution containing a fat-soluble substance" constituted by the present invention, examples of "aqueous solutions containing α-tocopherol and / or γ-tocotrienol" include aqueous solutions containing, for example, 3.3 µg / mL or greater, 99.9 µg / mL or greater or 339.6 µg / mL or greater α-tocopherol as well as 4.5 µg / mL or greater, 517.1 µg / mL or greater or 1964.4 µg / mL or greater γ-tocotrienol.
Upon obtaining such an "aqueous solution containing α-tocopherol and / or γ-tocotrienol" constituting the present invention, the solution may be combined with any of the super solubilizers referred to in the present invention. For example, NaTC or GZK₂ (at a content of 97.3% or greater) (Tokiwa Phytochemical Co., Ltd.) may be combined with the "method for the production of an aqueous solution containing a fat-soluble substance" constituted by the present invention.

In the event that, upon production of the "aqueous solution containing α-tocopherol and / or γ-tocotrienol" constituting the present invention, the super solubilizer which is combined is NaTC, NaTC can be added such that it comprises 0.5% (w/v) or greater of the total amount of "aqueous solution containing α-tocopherol and / or γ-tocotrienol" which is produced. Because the "method for the production of an aqueous solution containing a fat-soluble substance" constituted by the present invention increases the solubility of α-tocopherol and γ-tocotrienol in a concentration-dependent manner based on the amount of NaTC added, 1.0% (w/v) or greater or 2.0% (w/v) or greater NaTC can be added.
By combining NaTC as a super solubilizer, it is possible to produce an aqueous solution containing 3.3 µg / mL or greater, 6.0 µg / mL or greater, or 99.9 µg / mL or greater α-tocopherol and 4.5 µg / mL or greater, 34.2 µg / mL or greater, or 517.1 µg / mL or greater γ-tocotrienol.

In the event that, upon production of the "aqueous solution containing α-tocopherol and / or γ-tocotrienol" constituting the present invention, the super solubilizer which is combined is GZK₂, GZK₂ can be added such that it comprises 1.0% (w/w) or greater of the total amount of "aqueous solution containing α-tocopherol and / or γ-tocotrienol" which is produced. Because the "method for the production of an aqueous solution containing a fat-soluble substance" constituted by the present invention increases the solubility of α-tocopherol or γ-tocotrienol in a concentration-dependent manner based on the amount of GZK₂ added, 1.4% (w/v) or greater, 1.7% (w/v) or greater or 2.0% (w/v) or greater GZK₂ can be added.
By combining GZK₂ as a super solubilizer, it is possible to produce an aqueous solution containing 9.7 µg / mL or greater, 90.9 µg / mL or greater, or 339.6 µg / mL or greater α-tocopherol and 70.7 µg / mL or greater, 509.7 µg / mL or greater, or 1964.4 µg / mL or greater γ-tocotrienol.
Note, however, that since GZK₂ causes gelation at high concentrations, the amount of GZK₂ added should preferably not exceed 10% (w/v).

CD which is combined with the super solubilizer constituted by the present invention may be any conventionally known substance as long as it is useful in the production of the "aqueous solution containing α-tocopherol or γ-tocotrienol" which is constituted by the present invention, but γCD is preferable.
In particular, a "tocotrienol-containing oil-γCD complex" which includes α-tocopherol or γ-tocotrienol in γCD is preferable and an independently prepared substance may be used for this "tocotrienol-containing oil-γCD complex".

The "aqueous solution containing a fat-soluble substance" obtained via the present invention features high absorption of the fat-soluble substance contained in the aqueous solution through the skin and efficiently incorporates useful fat-soluble substances into the skin, including the epidermis and dermis. Therefore, the "aqueous solution containing a fat-soluble substance" obtained via the present invention can be used as an effective ingredient in cosmetics such as creams, lotions and cosmetic toners; food products such as jelly, yogurt, juice and chewable tablets as well as various pharmaceutical products.

Note that when producing cosmetics, food products or pharmaceutical products using the "aqueous solution containing a fat-soluble substance" constituted by the present invention, preservatives, perfumes or anti-oxidants which are safe for use in mammals, including humans, may be added as needed. Examples of preservatives include both natural plant extracts as well as chemically synthesized preservatives. Examples of such preservatives include plant extracts such as hinoki thiol, rosemary extract and eucalyptus extract as well as chemically synthesized preservatives such as p-hydroxybenzoic acid ester or the sodium salt thereof, phenoxyethanol and 1,3-butylene glycol. For Japan, examples include the preservatives specified in Appendix 3 of the Cosmetics Criteria promulgated in Ministry of Health and Welfare Notice No. 331 (dated September 29, 2000)

Examples of antioxidants include compounds with strong anti-oxidant properties such as astaxanthin, ginkgo biloba extract, carotenoids, sesame oil, tocotrienol, fullerene, vitamin C, vitamin E, eucalyptus extract and rosemary extract.
Examples of perfume agents include, for example, natural perfume agents such as terpene natural essential oil, as well as lavender, agarwood, ambergris and musk. Furthermore, floral fragrances such as rose, orchid, camellia, lotus, cherry etc. may be used as perfume agents. Moreover, synthetic perfumes produced via chemical synthesis such as anisaldehyde and acetophenone may be used.
A number of compounds have been listed above as examples of preservatives, anti-oxidants and perfume agents which can be added, but compounds other than those listed above may be used provided the substance is safe for use in mammals, including humans.

In the following section, the present invention shall be further described by way of examples, but the present invention is not limited to these examples.

### Example 1

### A. An aqueous solution containing curcumin

### <Specimen>

1) The curcumin-γCD complex CAVAMAX® W8 Curcumin (curcumin content of 10.5%, Wacker Chemical Ltd.) was used as a source of curcumin.
2) One of the following was used as a super solubilizer.
   (1) Sodium taurocholate (Wako Pure Chemical Industries, Ltd.)
   (2) Glycyrrhizic acid (Gurichinon GT1, 65% or greater content) (Tokiwa
      Phytochemical Co., Ltd.)
   (3) Soybean saponin (Saponin AZ-B, 84.1% content) (J-Oil Mills, Inc.)
   (4) Quillaja saponin (Kirayanin S-100, 25% quillaja extract) (Maruzen Pharmaceuticals, Ltd.)
   (5) Polysorbate 20 (Tokyo Chemical Industry Co., Ltd.)
   (6) Polysorbate 80 (Tokyo Chemical Industry Co., Ltd.)
   (7) Methylcellulose 15 (Wako Pure Chemical Industries, Ltd.)
   (8) Methylcellulose 50 (Wako Pure Chemical Industries, Ltd.)
   (9) Methylcellulose 100 (Wako Pure Chemical Industries, Ltd.)
   (10) Methylcellulose 400 (Wako Pure Chemical Industries, Ltd.)
   (11) Glycyrrhizin K₂ (97.3% or greater content) (Tokiwa Phytochemical Co., Ltd.)
   (12) Sodium glycocholate (Steraloids)

### <Preparation of the Aqueous Solution Containing Curcumin>

### A-1 Super Solubilizer: Sodium Taurocholate (NaTC)

### Preparation Method 1)

1450 µL of a 50 mM 2-morpholinoethanesulfonic acid (MES) buffer solution (containing 300 mM NaCl, 140 mM KSCN and 5 mM CaCl₂) or the aforementioned buffer solution into which a super solubilizer (0.83% NaTC or 1.66% NaTC) was dissolved was added to 49.6 mg of CAVAMAX^{®} W8 Curcumin (4 mg water content, 5.6 mg curcumin and 40 mg γCD) and sonication was performed for several seconds using an ultrasonic apparatus (Ikeda Scientific Co., Ltd.) and left to stand for 2 hours at 40°C.
Next, the mixture was filtered using a 0.2 µm filter and the curcumin concentration of the resulting solution was analyzed using an HPLC analyzer (Shimadzu, LC-2010C) under the conditions shown in Table 1 to obtain the solubility of curcumin.

As a comparison, in lieu of CAVAMAX^{®} W8 Curcumin, 5.6 mg of curcumin was combined with a 50 mM 2-morpholinoethanesulfonic acid (MES) buffer solution (containing 300 mM NaCl, 140 mM KSCN and 5 mM CaCl₂) or the aforementioned buffer solution into which a super solubilizer (0.83% (w/v) NaTC or 1.66% (w/v) NaTC) was dissolved and the resulting mixture was prepared as described above. The mixture was then filtered and the solution obtained was analyzed via HPLC as described above.

**[Table 1]**

| HPLC Conditions | |
|---|---|
| Column | Waters ; Sun Fire C18 5 um(4.6 mm I.D.x100 mm) |
| Mobile Phase | 10 mM NaH₂PO₄ (pH2.6)/MeOH = 30/70 |
| Flow Rate | 0.8 mL/min |
| Temperature | 40°C |
| Detection | UV 430 nm |
| Injection Volume | 1 µL |

The results showed that, as shown in Table 2, when NaTC was incorporated into CAVAMAX^{®} W8 Curcumin as a super solubilizer, the solubility of curcumin increased in a concentration-dependent manner and it was confirmed that an aqueous solution containing curcumin could be prepared.
On the other hand, when NaTC was combined with curcumin as a comparison, a sufficient increase in solubility was not observed.

**[Table 2]**

| Curcumin Concentration (µg / mL) | | | |
|---|---|---|---|
| NaTC concentration | 0% (w/v) | 0.83% (w/v) | 1.66% (w/v) |
| Using a Curcumin-γCD Complex | 0.57 | 269.2 | 723.3 |
| Using Curcumin | 3.95 | 35.35 | 219.2 |

### Preparation Method 2)

Unlike in Preparation Method 1) described above, sonication and heat treatment (40°C, left to stand) were not carried out and an aqueous solution containing curcumin was prepared.
2 mL each of ion-exchanged water or a solution produced by dissolving a super solubilizer (0.5% (w/v) NaTC, 1.0% (w/v) NaTC, 2.0% NaTC, or 4.0% NaTC) in ion-exchanged water was added to 12.4 mg CAVAMAX^{®} W8 Curcumin (1 mg water content, 1.4 mg curcumin and 10 mg γCD) and mixing by inversion was performed several times.
Next, the mixture was filtered using a 0.2 µm filter and the curcumin concentration of the resulting solution was analyzed using an HPLC analyzer (Shimadzu, LC-2010C) under the conditions shown in Table 1 to obtain the solubility of curcumin.
The results showed that, as shown in Table 3, even when sonication and heat treatment (40°C, left to stand) were not carried out, curcumin was sufficiently dissolved and an aqueous solution containing curcumin was successfully prepared. However, since filtration and analysis were performed immediately following mixing, it can be inferred that the curcumin contained in the solution had not yet reached equilibrium.

**[Table 3]**

| Curcumin Concentration (µg / mL) | | | | | |
|---|---|---|---|---|---|
| NaTC concentration | 0% (w/v) | 0.5% (w/v) | 1.0% (w/v) | 2.0% (w/v) | 4.0% (w/v) |
| Using a Curcumin-γCD Complex | 0.01 | 160.47 | 359.81 | 454.12 | 496.36 |

### A-2. Super Solubilizer: Glycyrrhizin (GZ)

1950 µL each of a 50 mM 2-morpholinoethanesulfonic acid (MES) buffer solution (containing 300 mM NaCl, 140 mM KSCN and 5 mM CaCl₂) or the aforementioned buffer solution into which a super solubilizer (3.5% (w/v) GZ) was dissolved were added to 49.6 mg of CAVAMAX^{®} W8 Curcumin (4 mg water content, 5.6 mg curcumin and 40 mg γCD) and sonication was performed for several seconds using an ultrasonic apparatus (Ikeda Scientific Co., Ltd.) and left to stand for 2 hours at 40°C.
Next, the mixture was filtered using a 0.2 µm filter and the curcumin concentration of the resulting solution was analyzed using an HPLC analyzer (Shimadzu, LC-2010C) under the conditions shown in Table 1 to obtain the solubility of curcumin.

As a comparison, in lieu of CAVAMAX^{®} W8 Curcumin, 5.6 mg of curcumin was combined with a 50 mM 2-morpholinoethanesulfonic acid (MES) buffer solution (containing 300 mM NaCl, 140 mM KSCN and 5 mM CaCl₂) or the aforementioned buffer solution into which a super solubilizer (3.5% (w/v) GZ) was dissolved, the resulting mixture was prepared as described above and left to stand and the solution obtained after filtration was analyzed using HPLC.

The results showed that, as shown in Table 4, when GZ was incorporated into CAVAMAX^{®} W8 Curcumin as a super solubilizer, the solubility of curcumin increased in a concentration-dependent manner and it was confirmed that an aqueous solution containing curcumin could be prepared.
On the other hand, when GZ was combined with curcumin as a comparison, a sufficient increase in solubility was not observed.

**[Table 4]**

| Curcumin Concentration (µg / mL) | | |
|---|---|---|
| GZ Concentration | 0% (w/v) | 3.50% (w/v) |
| Using a Curcumin-γCD Complex | 0.57 | 495.81 |
| Using Curcumin | 3.95 | 98.19 |

### A-3. Super Solubilizer: Soybean Saponin (SS)

### Preparation Method 1)

1950 µL each of ion-exchanged water or a solution produced by dissolving a super solubilizer (0.99% (w/v) SS, 1.98% (w/v) SS, or 3.97% (w/v) SS) in ion-exchanged water was added to 12.4 mg CAVAMAX^{®} W8 Curcumin (1 mg water content, 1.4 mg curcumin and 10 mg γCD) and sonication was performed for several seconds using an ultrasonic apparatus (Ikeda Scientific Co., Ltd.) and left to stand for 30 min at 40°C.
Next, the mixture was filtered using a 0.2 µm filter and the curcumin concentration of the resulting solution was analyzed using an HPLC analyzer (Shimadzu, LC-2010C) under the conditions shown in Table 1 to obtain the solubility of curcumin.
The results showed that, as shown in Table 5, when SS was incorporated into CAVAMAX^{®} W8 Curcumin as a super solubilizer, the solubility of curcumin increased in a concentration-dependent manner and it was confirmed that an aqueous solution containing curcumin could be prepared.

**[Table 5]**

| Curcumin Concentration (µg / mL) | | | | |
|---|---|---|---|---|
| SS Concentration | 0% (w/v) | 0.99% (w/v) | 1.98% (w/v) | 3.97% (w/v) |
| Using a Curcumin-γCD Complex | 0.57 | 427 | 468 | 617 |

### Preparation Method 2)

Unlike in Preparation Method 1) described above, the amount of CAVAMAX^{®} W8 Curcumin used was adjusted to 18.6 mg or 25 mg and combined with a solution made by dissolving a super solubilizer (3.97% (w/v) SS) in water which was then prepared as described above and left to stand.
In addition, the above mixture was filtered and analyzed using HPLC as described above.
The results showed that, as shown in Table 6, the solubility of curcumin increased in a concentration-dependent manner in accordance with the amount of CAVAMAX^{®} W8 Curcumin used and it was confirmed that an aqueous solution containing curcumin could be prepared.

**[Table 6]**

| SS Concentration | 3.97% (w/v) | 3.97% (w/v) | 3.97% (w/v) |
|---|---|---|---|
| Using a Curcumin-γCD Complex | 12.4 mg | 18.6 mg | 25 mg |
| Curcumin Concentration (µg / mL) | 617 | 847 | 1050 |

### A-4. Super Solubilizer: Quillaja Saponin (QS)

0 µL, 50 µL, 100 µL, 200 µL or 1000 µL quillaja saponin (in a 10% ethanol solution) was added to 6.2 mg CAVAMAX^{®} W8 Curcumin (0.5 mg water content, 0.7 mg curcumin and 5 mg γCD) as a super solubilizer, the total volume of the mixture was adjusted to 2 mL using a 10% aqueous solution of ethanol and sonication was performed for several seconds using an ultrasonic apparatus (Ikeda Scientific Co., Ltd.) and left to stand for 1 hour at 40°C.
Next, the mixture was filtered using a 0.2 µm filter and the curcumin concentration of the resulting solution was analyzed using an HPLC analyzer (Shimadzu, LC-2010C) under the conditions shown in Table 1 to obtain the solubility of curcumin.

As a comparison, 0.7 mg of curcumin was used in lieu of CAVAMAX^{®} W8 Curcumin, 0, 200 or 1000 µL quillaja saponin was added as a super solubilizer, the total volume of the mixture was adjusted to 2 mL using a 10% aqueous solution of ethanol and the resulting mixture was prepared as described above. The mixture was then allowed to stand, was filtered and the resulting solution was analyzed using HPLC.
The results showed that, as shown in Table 7, when QS was incorporated into CAVAMAX^{®} W8 Curcumin as a super solubilizer, the solubility of curcumin increased in a concentration-dependent manner and it was confirmed that an aqueous solution containing curcumin could be prepared. On the other hand, when QS was combined with curcumin as a comparison, a sufficient increase in solubility was not observed.

**[Table 7]**

| Curcumin Concentration (µg / mL) | | | | | |
|---|---|---|---|---|---|
| Amount of QS Added | 0 µL | 50 µL | 100 µL | 200 µL | 1000 µL |
| Using a Curcumin-γCD Complex | 5.01 | 130.81 | 254.54 | 320.76 | 386.02 |
| Using Curcumin | 0.02 | - | - | 16.81 | 122.96 |

### A-5. Super Solubilizer: Polysorbate

A solution created by dissolving polysorbate 20 or polysorbate 80 in water (polysorbate 20 aqueous solution or polysorbate 80 aqueous solution) as a super solubilizer was added to 12.4 mg CAVAMAX^{®} W8 Curcumin (1 mg water content, 1.4 mg curcumin and 10 mg γCD) at the concentrations shown in Table 8 such that the total volume was brought to 2 mL. This mixture was then sonicated for several seconds using an ultrasonic apparatus (Ikeda Scientific Co., Ltd.) and left to stand for 10 min at 40°C.
Next, the mixture was filtered using a 0.2 µm filter and the curcumin concentration of the resulting solution was analyzed using an HPLC analyzer (Shimadzu, LC-2010C) under the conditions shown in Table 1 to obtain the solubility of curcumin.

As a comparison, 2 mL ion-exchanged water (in lieu of polysorbate aqueous solution) was added to 1.4 mg of curcumin (in lieu of CAVAMAX^{®} W8 Curcumin), and processed as described above. The resulting mixture was left to stand and filtered and the resulting solution was analyzed using HPLC.

**[Table 8]**

| Super Solubilizer | Curcumin Concentration (µg / mL) |
|---|---|
| None (comparison) | 0.01 |
| 1% (w/v) aqueous solution of polysorbate 80 | 145.31 |
| 1% (w/v) aqueous solution of polysorbate 20 | 230.12 |
| +1% (w/v) aqueous solution of polysorbate 80 | |
| 2% (w/v) aqueous solution of polysorbate 20 | 226.46 |
| 2% (w/v) aqueous solution of polysorbate 80 | 198.94 |

### A-6 Super Solubilizer: Methylcellulose

A solution created by dissolving methylcellulose 15, methylcellulose 50, methylcellulose 100, or methylcellulose 400 in water (methylcellulose 15 aqueous solution, methylcellulose 50 aqueous solution, methylcellulose 100 aqueous solution or methylcellulose 400 aqueous solution) as a super solubilizer was added to 12.4 mg CAVAMAX^{®} W8 Curcumin (1 mg water content, 1.4 mg curcumin and 10 mg γCD) at the concentrations shown in Table 9 such that the total volume was brought to 2 mL. This mixture was then mixed by inversion.
Immediately following inversion, the mixture was filtered using a 0.2 µm filter and the curcumin concentration of the resulting solution was analyzed using an HPLC analyzer (Shimadzu, LC-2010C) under the conditions shown in Table 1 to obtain the solubility of curcumin.

As a comparison, 2 mL ion-exchanged water (in lieu of methylcellulose 400 aqueous solution) was added to 1.4 mg of curcumin (in lieu of CAVAMAX^{®} W8 Curcumin), and processed as described above. The resulting mixture was left to stand and filtered and the resulting solution was analyzed using HPLC. The results are shown in Table 9.

**[Table 9]**

| Super Solubilizer | Curcumin Concentration (µg / mL) |
|---|---|
| None (comparison) | 0.01 |
| 0.5%(w/v)MC15 | 11.77 |
| 1.0%(w/v) MC15 | 16.18 |
| 0.5%(w/v)MC50 | 10.41 |
| 1.0%(w/v)MC50 | 15.36 |
| 0.5%(w/v)MC100 | 9.79 |
| 1.0%(w/v)MC100 | 12.72 |
| 0.5%(w/v)MC400 | 9.09 |
| 1.0%(w/v)MC400 | 10.33 |

### A-7. Super Solubilizer: Glycyrrhizin K₂ (GZK₂)

0 mg or 33 mg of GZK₂ was mixed into 24 mg of CAVAMAX^{®} W8 Curcumin (curcumin 3 mg) as a super solubilizer and 3 mL purified water was added. The resulting mixture was then sonicated for 1 hour using an ultrasonic apparatus (Ikeda Scientific Co., Ltd.).
Next, the mixture was filtered using a 0.2 µm filter and the curcumin concentration of the resulting solution was analyzed using an HPLC analyzer (Shimadzu, LC-2010C) under the conditions shown in Table 1 to obtain the solubility of curcumin.

The results showed that, as shown in Table 10, when GZK₂ was incorporated into CAVAMAX^{®} W8 Curcumin as a super solubilizer, the solubility of curcumin increased in a concentration-dependent manner and it was confirmed that an aqueous solution containing curcumin could be prepared.

**[Table 10]**

| Curcumin Concentration (µg / mL) | | |
|---|---|---|
| Amount of GZK₂ Added | 0 mg | 33 mg |
| Using a Curcumin-γCD Complex | 0.21 | 442.91 |

### A-8. Super Solubilizer: Na Glycocholate

A solution created by dissolving Na glycocholate in water (Na glycocholate solution) as a super solubilizer was added to 12.4 mg CAVAMAX^{®} W8 Curcumin (1 mg water content, 1.4 mg curcumin and 10 mg γCD) at the concentrations shown in Table 11 such that the total volume was brought to 2 mL.
Immediately following mixing, the mixture was filtered using a 0.2 µm filter and the curcumin concentration of the resulting solution was analyzed using an HPLC analyzer (Shimadzu, LC-2010C) under the conditions shown in Table 1 to obtain the solubility of curcumin.

As a comparison, 2 mL of 200% Na glycocholate aqueous solution was added to 1.4 mg of curcumin (in lieu of CAVAMAX^{®} W8 Curcumin) as a super solubilizer, and processed as described above. The resulting mixture was left to stand and filtered and the resulting solution was analyzed using HPLC. The results are shown in Table 11.

**[Table 11]**

| Curcumin Concentration (µg / mL) | | | | |
|---|---|---|---|---|
| Amount of Na Glycocholate Added | 0%(w/v) | 0.5%(w/v) | 1.0%(w/v) | 2.0%(w/v) |
| Using a Curcumin-γCD Complex | 0.01 | 273.34 | 497.87 | 567.42 |
| Using Curcumin | - | - | - | 12.31 |

The results of A-1 to A-8 show that the combination of a CD complex including curcumin with a super solubilizer such as sodium taurocholate, glycyrrhizic acid, soybean saponin, quillaja saponin, polysorbate aqueous solution, methylcellulose, GZK₂ or Na glycocholate is particularly effective for preparing an aqueous solution containing curcumin.

### Example 2

### <Confirmation Testing>

The incorporation of an aqueous solution containing a high concentration of curcumin which was obtained in the same manner as Example 1 into skin (dermal absorption) was checked.

### <Specimen>

### 1) Test Specimen

Test specimen: 33 mg of GZK₂ was mixed into 24 mg of CAVAMAX^{®} W8 Curcumin (curcumin-γCD complex, containing 3 mg of curcumin) and the resulting mixture was stirred after adding 3 mL of ultra-pure water. The resulting mixture was then filtered using a 0.2 µm filter and the filtrate was used as a test specimen.

### 2) Comparative Specimen

Comparative specimen 1: 3 mL of ultra-pure water was added to 24 mg of CAVAMAX^{®} W8 Curcumin (curcumin-γCD complex, containing 3 mg of curcumin) and sonicated for 30 minutes. The resulting mixture was used as a comparative specimen (curcumin-γCD complex suspension).

### <Skin Incorporation Testing>

The "LabCyte EPI-MODEL 12" 3-dimensional cultured human skin model (Japan Tissue Engineering Co., Ltd.) was used to evaluate the incorporation of curcumin into the skin (dermal absorption).
That is, after incubating (37°C, 5% CO₂) the 3-dimensional cultured human skin model overnight in assay medium, the model was exposed to the 200 µL of the aforementioned test specimen or comparative specimen and incubated (37°C, 5% CO₂) for 6 hours.
Next, the exposed test specimen or comparative specimen was washed with 0.1 M PBS (pH 7.2) and the curcumin contained in the 3-dimensional cultured human skin model was extracted with chloroform / methanol (1:1) and quantified using HPLC under the conditions shown in Table 12.
The results showed that, as shown in Table 13, the test specimen prepared by combining curcumin-γCD complex with GZK₂ as a super solubilizer was incorporated into the skin (showed dermal absorption) at a rate approximately 40 times that of the comparative specimen, constituting a significant increase.

**[Table 12]**

| | |
|---|---|
| Column | Waters ; Sun Fire C18 5 um(4.6 mm I.D. x100 mm) |
| Mobile Phase | 1.67% aqueous solution of citric acid : THF = 60 : 40 |
| Flow Rate | 0.35 mL/min |
| Temperature | 35°C |
| Detection | UV 425 nm |
| Injection Volume | 5 µL |

**[Table 13]**

| Curcumin Concentration (µg / well) | | |
|---|---|---|
| Test Specimen | Curcumin-γCD Complex + GZK₂ Solution | 3.94 |
| Comparative Specimen 1 | Curcumin-γCD Complex Suspension | 0.10 |

### Example 3

### B. Aqueous Solution Containing CoQ10

### <Specimen>

1) The CoQ10-γCD complex CAVAMAX® W8 CoQ10 (21.3% CoQ10 content, Wacker Chemical Ltd.) was used.
2) Super Solubilizer
NaTC (Wako Pure Chemical Industries, Ltd.) or GZK₂ (97.3% or greater content, Tokiwa Phytochemical Co., Ltd.) was used as a super solubilizer.

### <Preparation of the Aqueous Solution Containing CoQ10>

### B-1. Super Solubilizer Sodium Taurocholate (NaTC)

1450 µL each of a 50 mM 2-morpholinoethanesulfonic acid (MES) buffer solution (containing 300 mM NaCl, 140 mM KSCN and 5 mM CaCl₂) or the aforementioned buffer solution into which a super solubilizer (0.42% (w/v) NaTC, 0.83% (w/v) NaTC or 1.66% (w/v) NaTC) was dissolved were added to 57 mg of CAVAMAX^{®} W8 CoQ10 (5 mg water content, 12.14 mg CoQ10 and 40 mg γCD) and sonication was performed for several seconds using an ultrasonic apparatus (Ikeda Scientific Co., Ltd.) and left to stand for 2 hours at 40°C.
Next, the mixture was filtered using a 0.2 µm filter and the CoQ10 concentration of the resulting solution was analyzed using an HPLC analyzer (Shimadzu, LC-2010C) under the conditions shown in Table 14 to obtain the solubility of CoQ10.

As a comparison, 2 mL of 50 mM 2-morpholinoethanesulfonic acid (MES) buffer solution (containing 300 mM NaCl, 140 mM KSCN and 5 mM CaCl₂) (in lieu of NaTC) was added to 12.14 mg of CoQ10 (in lieu of CAVAMAX^{®} W8 CoQ10), and the mixture was prepared as described above. The resulting mixture was left to stand and filtered and the resulting solution was analyzed using HPLC.

**[Table 14]**

| HPLC Conditions | |
|---|---|
| Column | Phenomenex; Luna 5u C18 (2) 100A (4.6 mm I.D.x250 mm) |
| Mobile Phase | MeOH : EtOH = 60 : 40 |
| Flow Rate | 1.0 mL/min |
| Temperature | 35°C |
| Detection | UV 275 nm |
| Injection Volume | 10 µL |

The results showed that, as shown in Table 15, when NaTC was incorporated into CAVAMAX^{®} W8 CoQ10 as a super solubilizer, the solubility of CoQ10 increased in a concentration-dependent manner and it was confirmed that an aqueous solution containing CoQ10 could be prepared. On the other hand, when NaTC was combined with CoQ10 as a comparison, a sufficient increase in solubility was not observed.

**[Table 15]**

| CoQ10 Concentration (µg / mL) | | | | |
|---|---|---|---|---|
| NaTC concentration | 0%(w/v) | 0.42%(w/v) | 0.83%(w/v) | 1.66%(w/v) |
| Using a CoQ10-γCD Complex | 0 | 125.32 | 389.53 | 504.71 |
| Using CoQ10 | 0 | 0 | 0 | 1.52 |

### B-2. Super Solubilizer: Glycyrrhizin K₂ (GZK₂)

0 mg, 6.2 mg, 12.5 mg, 25 mg or 50 mg of GZK₂ was mixed into 60 mg of CAVAMAX^{®} W8 CoQ10 (containing 12 mg of CoQ10) as a super solubilizer and 3 mL purified water was added. The resulting mixture was then sonicated for 1 hour using an ultrasonic apparatus (Ikeda Scientific Co., Ltd.).
Next, the mixture was filtered using a 0.2 µm filter and the CoQ10 concentration of the resulting solution was analyzed using an HPLC analyzer (Shimadzu, LC-2010C) under the conditions shown in Table 14 to obtain the solubility of CoQ10.

As a comparison, GZK₂ was incorporated into 12 mg of CoQ10 as a super solubilizer and the mixture was prepared as described above. The resulting mixture was left to stand and filtered and the resulting solution was analyzed using HPLC.

The results showed that, as shown in Table 16, when GZK₂ was incorporated into CAVAMAX^{®} W8 CoQ10 as a super solubilizer, the solubility of CoQ10 increased in a concentration-dependent manner and it was confirmed that an aqueous solution containing CoQ10 could be prepared. On the other hand, when GZK₂ was combined with CoQ10 as a comparison, a sufficient increase in solubility was not observed.

**[Table 16]**

| CoQ10 Concentration (µg / mL) | | | | | |
|---|---|---|---|---|---|
| Amount of GZK₂ Added | 0 mg | 6.2 mg | 12.5 mg | 25 mg | 50 mg |
| Using a CoQ10-γCD Complex | 2.1 | 134.5 | 364.0 | 964.5 | 1749.0 |
| Using CoQ10 | 0.2 | 3.6 | 9.1 | 3.9 | 3.9 |

### Example 4

### <Confirmation Testing>

The incorporation of an aqueous solution containing a high concentration of CoQ10 which was obtained in the same manner as Example 3 into skin (dermal absorption) was checked.

### <Specimen>

### 1) Test Specimen

Test specimen: 33 mg of GZK₂ was mixed into 60 mg of CAVAMAX^{®} W8 CoQ10 (containing 12 mg of CoQ10) and the resulting mixture was stirred after adding 3 mL of ultra-pure water. The resulting mixture was then filtered using a 0.2 µm filter and the filtrate was used as a test specimen.

### 2) Comparative Specimen

Comparative Specimen 1: 10 mL of ultra-pure water was added to 10 mg of CoQ10 (BioQ10, Mitsubishi Gas Chemical Co., Inc.) and sonicated for 1 hour in an ultrasonic apparatus (produced by Ikeda Scientific). The resulting mixture was used as Comparative Specimen 1 (CoQ10 suspension).
Comparative Specimen 2: 10 mL of ultra-pure water was added to 50 mg of CAVAMAX^{®} W8 CoQ10 (containing 10 mg of CoQ10) and sonicated for 1 hour in an ultrasonic apparatus (produced by Ikeda Scientific). The resulting mixture was used as Comparative Specimen 2 (CoQ10-γCD complex suspension).
Comparative Specimen 3: 33 mg of GZK₂ was added to 3 mg of CoQ10 (BioQ10, Mitsubishi Gas Chemical Co., Inc.) as a super solubilizer and an additional 3 mL of ultra-pure water was added. The resulting mixture was sonicated for 1 hour in an ultrasonic apparatus (produced by Ikeda Scientific) and used as Comparative Specimen 3 (CoQ10 + GZK₂ suspension).
Comparative Specimen 4: Commercially available ENERGISOME JQ10 (Sederma) was used as Comparative Specimen 4 (CoQ10 liposomal formulation).

### <Skin Incorporation Testing>

The "LabCyte EPI-MODEL 12" 3-dimensional cultured human skin model (Japan Tissue Engineering Co., Ltd.) was used to evaluate the incorporation of CoQ10 into the skin (dermal absorption).
That is, after incubating (37°C, 5% CO₂) the 3-dimensional cultured human skin model overnight in assay medium, the model was exposed to the 200 µL of the aforementioned test specimen or Comparative Specimens 1 - 4 and incubated (37°C, 5% CO₂) for 6 hours.
Next, the exposed test specimen or Comparative Specimen 1 - 4 was washed with 0.1 M PBS (pH 7.2) and the CoQ10 contained in the 3-dimensional cultured human skin model was extracted with chloroform / methanol (1:1) and quantified using LL-MS under the conditions shown in Table 17.
The results showed that, as shown in Table 18, the test specimen prepared by combining CAVAMAX^{®} W8 CoQ10 with GZK₂ as a super solubilizer was incorporated into the skin (showed dermal absorption) at a rate approximately 20 - 40 times that of Comparative Specimens 1 - 4, constituting a significant increase.
Therefore, these results show that an aqueous solution, constituted by the present invention, containing a high concentration of CoQ10 such as the test specimen can be used in the production of cosmetics which incorporate CoQ10 at high concentrations in skin (show a high rate of dermal absorption) with the composition shown in Table 19, for example.

**[Table 17]**

| LC-MS Analysis Conditions | |
|---|---|
| Column | Phenomenex ; Luna 5u C18(2) 100^{a} (4.6 mm I.D.x150 mm) |
| Mobile Phase | 0.5% Formic acid in (Acetonitrile : Isopropanol = 8 : 7) |
| | +0.1% Trisodium citrate aq. (1 mg/mL) |
| Flow Rate | 0.2 mL/min |
| Temperature | 35°C |
| Detection | MS; ESI (+) m/z 885.9 ([M+Na]+) |
| Injection Volume | 20 µL |

**[Table 18]**

| CoQ10 Concentration (µg / well) | | |
|---|---|---|
| Control | PBS | 0.2 |
| Test Specimen | CoQ10-γCD complex + GZK₂ Solution | 17.3 |
| Comparative Specimen 1 | CoQ10 Suspension | 0.5 |
| Comparative Specimen 2 | CoQ10-γCD Complex Suspension | 0.4 |
| Comparative Specimen 3 | CoQ10 + GZK₂ Suspension | 0.7 |
| Comparative Specimen 4 | CoQ10 Liposomal Formulation | 0.9 |

**[Table 19]**

| Ingredients | Amount of Ingredient (%) (w/v) |
|---|---|
| Stearic Acid | 2.00 |
| PEG-40 Stearate | 2.00 |
| Glyceryl Stearate (SE) | 5.00 |
| Behenyl Alcohol | 2.00 |
| Squalene | 12.00 |
| Jojoba Oil | 4.00 |
| Dimethicone | 0.20 |
| Glycerin | 7.00 |
| Test Specimen | 30.00 (0.03 as CoQ10) |
| Preservative | As Needed |
| Water | Remainder |
| Total Amount | 100.00 |

Furthermore, the test specimen and Comparative Specimens 1 - 3 were filtered using a 0.2 µm filter and the CoQ10 concentration of the resulting solution was analyzed using an HPLC analyzer (Shimadzu, LC-2010C) under the conditions shown in Table 14 to obtain the solubility of CoQ10.
The results showed that, as shown in Table 20, when GZK₂ was incorporated into CAVAMAX^{®} W8 CoQ10 as a super solubilizer, the solubility of CoQ10 increased versus Comparative Examples 1 - 3 in a concentration-dependent manner and it was confirmed that an aqueous solution containing CoQ10 could be prepared.

**[Table 20]**

| CoQ10 Concentration (µg / mL) | | |
|---|---|---|
| Test Specimen | CoQ10-γCD complex + GZK₂ Solution | 1170.8 |
| Comparative Specimen 1 | CoQ10 Suspension | 0.3 |
| Comparative Specimen 2 | CoQ10-γCD Complex Suspension | 2.9 |
| Comparative Specimen 3 | CoQ10 + GZK₂ Suspension | 3.9 |

### Example 5

### C. Aqueous Solution Containing α-lipoic Acid

### <Specimen>

### 1) α-Lipoic Acid

The α-lipoic acid-γCD complex R-α-lipoic acid CD (11.4% R-α-lipoic acid content, Cyclo Chem Bio) was used.

### 2) Super Solubilizer

Na taurocholate (Wako Pure Chemical Industries, Ltd.) was used as a super solubilizer.

### <Preparation of the Aqueous Solution of α-Lipoic Acid>

### C-1 Super Solubilizer: Sodium taurocholate (NaTC)

200 mg of R-α-lipoic acid CD was added to 5 mL of 0%, 1% (w/v), 2% (w/v) or 3% (w/v) NaTC aqueous solution and the resulting mixture was sonicated for 5 minutes in an ultrasonic apparatus (Ikeda Scientific Co., Ltd.) such that even dispersal of the contents was attained.
Next, the mixture was filtered using a 0.2 µm filter and the R-α-lipoic acid concentration of the resulting solution was analyzed using an HPLC analyzer (Shimadzu, LC-2010C) under the conditions shown in Table 21 to obtain the solubility of R-α-lipoic acid.
The results showed that, as shown in Table 22, when NaTC was incorporated into R-α-lipoic acid CD as a super solubilizer, the solubility of α-lipoic acid increased in a concentration-dependent manner and it was confirmed that an aqueous solution containing α-lipoic acid could be prepared.

**[Table 21]**

| HPLC Conditions | |
|---|---|
| Column | CHIRALPAK AD-RH (4.6 mm I.D.x150 mm) |
| Mobile Phase | 25 mM Phosphate buffer (pH3.5) :CH₃CN = 70:30 |
| Flow Rate | 0.6 mL/min |
| Temperature | 25°C |
| Detection | UV 215 nm |
| Injection Volume | 10 µL |

**[Table 22]**

| α-Lipoic Acid Concentration ( mg / mL) | | | | |
|---|---|---|---|---|
| NaTC concentration | 0% (w/v) | 1.0%(w/v) | 2.0%(w/v) | 3.0%(w/v) |
| α-Lipoic Acid Concentration | 0.67 | 0.98 | 1.02 | 1.43 |

### Example 6

### D. Aqueous Solution Containing Retinol

### <Specimen>

### 1) Retinol

The retinol-γCD complex CAVAMAX® W8 Retinol (6.5% retinol content, Wacker Chemical Ltd.) was used.

### 2) Super Solubilizer

Glycyrrhizin K₂ (97.3% or greater content) (Tokiwa Phytochemical Co., Ltd.) was used as a super solubilizer.

### <Preparation of the Aqueous Solution Containing Retinol>

### D-1. Super Solubilizer: Glycyrrhizin K₂ (GZK₂)

0 mg, 3 mg, 7 mg, 17 mg, 35 mg, 139 mg or 347 mg GZK₂ was incorporated in 36 mg of CAVAMAX^{®} W8 Retinol (containing 1.8 mg of retinol). 3mL of water was added to the resulting mixture and the mixture was then sonicated for 1 hour using an ultrasonic apparatus (Ikeda Scientific Co., Ltd.).
Next, the mixture was filtered using a 0.2 µm filter and the retinol concentration of the resulting solution was analyzed using an HPLC analyzer (Shimadzu, LC-2010C) under the conditions shown in Table 23 to obtain the solubility of retinol.
The results showed that, as shown in Table 24, when GZK₂ was incorporated into CAVAMAX^{®} W8 Retinol as a super solubilizer, the solubility of retinol increased in a concentration-dependent manner and it was confirmed that an aqueous solution containing retinol could be prepared.

**[Table 23]**

| HPLC Conditions | |
|---|---|
| Column | Phenomenex; Luna 5u C18(2) 100A (4.6 mm I.D. x150 mm) |
| Mobile Phase | MeOH:H₂0 = 90:10 |
| Flow Rate | 0.8 mL/min |
| Temperature | 35°C |
| Detection | UV 325 nm |
| Injection Volume | 5 µL |

**[Table 24]**

| Retinol Concentration (µg / mL) | | | | | | | |
|---|---|---|---|---|---|---|---|
| Amount of GZK₂ Added | 0 mg | 3 mg | 7 mg | 17 mg | 35 mg | 139 mg | 347 mg |
| Retinol Concentration | 0.7 | 27.9 | 90.0 | 357.1 | 389.4 | 439.2 | 443.1 |

### Example 7

### E. Aqueous Solution Containing Ferulic Acid

### <Specimen>

### 1) Ferulic Acid

Ferulic acid (Tsuno Co., Ltd.) was used.

### 2) CD

M-αCD (90% or greater, Fuji Nihon Seito) or the αCD source CAVAMAX^{®} W6 Food (99.8%, Wacker Chemical Ltd.) was used.

### 3) Super Solubilizer

Na taurocholate (NaTC) (Wako Pure Chemical Industries, Ltd.) was used as a super solubilizer.

<Preparation of the Aqueous Solution Containing Ferulic Acid> E-1. Super Solubilizer: Na taurocholate (NaTC) 100 mg of ferulic acid and 0.1 g of M-αCD (5% (w/v)) or 100 mg ferulic acid and 0.1 g of αCD (5% (w/v)) were added to ion-exchanged water. NaTC was then added as a super solubilizer such that its concentration was 0%, 0.5% (w/v), 1% (w/v) or 2% (w/v) and the entire volume was brought to 2 mL. It is believed that at this point the ferulic acid and CD formed a complex in the aqueous solution.
Next, the mixture was filtered using a 0.2 µm filter, the filtrate was placed in a spectrophotometer and the absorbance was measured at a wavelength of 316 nm.
The results showed that, as shown in Table 25, when NaTC was incorporated as a super solubilizer, the solubility of ferulic acid increased in a concentration-dependent manner and it was confirmed that an aqueous solution containing ferulic acid could be prepared.

**[Table 25]**

| Ferulic Acid Concentration ( mg / mL) | | | | |
|---|---|---|---|---|
| NaTC concentration | 0%(w/v) | 0.5%(w/v) | 1.0%(w/v) | 2.0%(w/v) |
| Using 5% (w/v) M-αCD | 14.95 | 15.7 | 16.42 | 18.09 |
| Using 5% (w/v) αCD | 15.69 | - | 0.10 | 21.24 |

### Example 8

### F. Aqueous Solution Containing Isoflavone

### <Specimen>

### 1) Isoflavone

AglyMax^{®}-30 (34.7% total isoflavone aglycone, Nichimo Biotics) was used.

### 2) Isoflavone-γCD Complex

Aglymax^{®}-30 (34.7% total isoflavone aglycone, Nichimo Biotics) and CAVAMAX® W8 Food (98% or greater γCD, Wacker Chemical Ltd.) were added to a mortar in a 20 : 80 (w/w) ratio and after adding a small amount of water and kneading the mixture, lyophilization was carried out. As a result, an isoflavone-γCD complex containing 7% (w/w) isoflavone aglycone was obtained.

### 3) Super Solubilizer

Methylcellulose 400 (Wako Pure Chemical Industries, Ltd.), soybean saponin (saponin AZ-B, 84.1% content) (J-Oil Mills, Inc.) or glycyrrhizic acid (Gurichinon GT1, 65% or greater content) (Tokiwa Phytochemical Co., Ltd.) was added as a super solubilizer.

### <Preparation of the Aqueous Solution Containing Isoflavone>

### F-1. Super Solubilizer: Methyl Cellulose

100 mg AglyMax^{®}-30 (containing 34.7 mg isoflavone aglycone) was added to 50 mL of ion-exchanged water and 1N of NaOH was added dropwise to dissolve the solute in an alkaline solution and 300 mg of βCD and 100 mg of γCD were added in the combination(s) shown in Table 26.
Furthermore, after adding 500 mg MC400 (methylcellulose 400) the mixture was re-acidified with hydrochloric acid. Isoflavone aglycone content when MC400 and CD were added was 0.068% (w/w). This mixture was left to stand at room temperature for 2 hours and no precipitation was observed.
Furthermore, after standing for 2 hours, the test specimen was placed in a test tube and stirred via shaking for 2 minutes. The suspension thus obtained was placed in a spectrophotometer cell and absorbance was quantified at 660nm to obtain turbidity. The results showed that, as shown in Table 26, turbidity was minimal when super solubilizer was combined with βCD, when super solubilizer was combined with γCD and when super solubilizer was combined with both βCD and γCD and an increase in solubility was not observed.

**[Table 26]**

| | | | | | | |
|---|---|---|---|---|---|---|
| MC400 (500 mg) | - | + | - | + | + | + |
| βCD (300 mg) | - | - | + | + | + | - |
| γCD (100 mg) | - | - | + | + | - | + |
| Turbidity (A660 nm) | 2.799 | 1.922 | 0.435 | 0.2 | 0.855 | 1.755 |

### F-2. Super Solubilizer: Soybean Saponin (SS)

150 mg isoflavone-γCD complex (7% isoflavone aglycone, 93% γCD) and 50 mg, 100 mg, 200 mg or 500 mg of SS were mixed into 50 mL of ion-exchanged water to obtain a solution.
Measurements of the turbidity (660 nm) of the resulting solution showed that, as shown in Table 27, when SS is combined with isoflavone-γCD complex, the solubility of isoflavone increased in a concentration-dependent manner and it was confirmed that an aqueous solution containing isoflavone could be prepared.

**[Table 27]**

| Turbidity (660 nm) | | | | |
|---|---|---|---|---|
| Amount of SS Added | 50 mg | 100 mg | 200 mg | 500 mg |
| Using an Isoflavone-γCD Complex | 1.906 | 1.22 | 0.426 | 0.491 |

### F-3. Super Solubilizer: Glycyrrhizin (GZ)

150 mg isoflavone-γCD complex (20% isoflavone, 80% γCD, includes 10.4 mg of isoflavone aglycone) and 50 mg or 100 mg of GZ were mixed into 50 mL of ion-exchanged water to obtain a solution.
Measurements of the turbidity (660 nm) of the resulting solution showed that, as shown in Table 28, when isoflavone-γCD complex is used and GZ is used as a super solubilizer, the solubility of isoflavone increased in a concentration-dependent manner and it was confirmed that an aqueous solution containing isoflavone could be prepared. On the other hand, when only isoflavone was used, a sufficient increase in solubility was not observed relative to when isoflavone-γCD complex was used, even when GZ was used.

As a comparison, a mixture of 30 mg AglyMax^{®}-30 (containing 10.4 mg isoflavone aglycone; used in lieu of isoflavone-γCD complex) and super solubilizer was prepared and turbidity (660 nm) was measured under the same conditions as above.

**[Table 28]**

| Turbidity (660 nm) | | |
|---|---|---|
| Amount of SS Added | 50 mg | 100 mg |
| Using an Isoflavone-yCD Complex | 1.469 | 0.627 |
| Using Isoflavone | 1.01 | 1.739 |

### Example 9

### G. Aqueous Solution Containing Rose Polyphenol

### <Specimen>

### 1) Rose Polyphenol

ROSE CRYSTA^{®}-70 (70% or greater rose polyphenol content, ToyoHakko Co., Ltd.) was used.

### 2) CD

The αCD source CAVAMAX® W6 Food (99.8%, Wacker Chemical Ltd.) was used.

### 2) Super Solubilizer

Na taurocholate (NaTC) (Wako Pure Chemical Industries, Ltd.) was used as a super solubilizer.

<Preparation of the Aqueous Solution Containing Rose Polyphenol> G-1. Super Solubilizer: Na Taurocholate (NaTC) 10 mg of ROSE CRYSTA^{®}-70 was suspended in 1 mL of ion-exchanged water and 50 mg of αCD (0.5% (w/v)) and 10 mg of NaTC (0.1% (w/v)) was added to the suspension. This mixture was left to stand at room temperature for 2 hours and no precipitation was observed.
Furthermore, after standing for 2 hours, the test specimen was placed in a test tube and stirred via shaking for 2 minutes. The suspension thus obtained was placed in a spectrophotometer cell and absorbance was quantified at 660 nm to obtain turbidity. The results verified, as shown in Table 29, that when a super solubilizer is combined with αCD, turbidity decreases and solubility is improved.

**[Table 29]**

| | | | |
|---|---|---|---|
| 0.1% (w/v) NaTC | - | - | + |
| 0.5% (w/v) αCD | - | + | + |
| Turbidity (A660 nm) | 0.97 | 0.74 | 0.42 |

### Example 10

### H. Aqueous Solution Containing Caffeic Acid Phenethyl Ester (CAPE)

### <Specimen>

### 1) CAPE-γCD Complex

CAPE (98% or greater content, Sigma-Aldrich Co., Ltd.) and CAVAMAX® W8 Food (98% or greater γCD, Wacker Chemical Ltd.) were used to create a preparation.
That is, 1.7 mL of 150 mM CAPE / DMSO solution was added dropwise to 1000 mL of 50 mM γCD aqueous solution while stirring with a stirrer at room temperature. Next, the resulting mixture was filtered with a 0.2 µm filter and the solid thus obtained was washed with water and dried under reduced pressure. As a result, a CAPE-γCD complex containing 14% (w/v) CAPE was obtained as an off-white powder.

### 2) Super Solubilizer

Na taurocholate (NaTC) (Wako Pure Chemical Industries, Ltd.) was used as a super solubilizer.

### <Preparation of the Aqueous Solution Containing CAPE>

### H-1. Super Solubilizer: Sodium Taurocholate (NaTC)

5 mL of water or a solution prepared by dissolving a super solubilizer (1.0% (w/v) NaTC) in water was added to 10 mg CAPE-γCD complex (1.4 mg CAPE and 8.6 mg γCD) and the resulting mixture was sonicated for 3 minutes in an ultrasonic apparatus (Ikeda Scientific Co., Ltd.) such that even dispersal of the contents was attained.
Next, the mixture was filtered using a 0.2 µm filter and the CAPE concentration of the resulting solution was analyzed using an HPLC analyzer (Shimadzu, LC-2010C) under the conditions shown in Table 30 to obtain the solubility of CAPE.

As a comparison, water or a solution prepared by dissolving a super solubilizer (1.0% (w/v) NaTC) in water was combined with 1.4 mg of CAPE (in lieu of CAPE-γCD complex) and the mixture was prepared as described above. The resulting mixture was left to stand and filtered and the resulting solution was analyzed using HPLC.

**[Table 30]**

| HPLC Conditions | |
|---|---|
| Column | Waters ; Sun Fire C18 5 um (4.6 mm I.D. x 100 mm) |
| Mobile Phase | 0.1% formic acid aqueous solution : CH₃CN = 50 : 50 |
| Flow Rate | 0.4 mL/min |
| Temperature | 35°C |
| Detection | UV 320 nm |
| Injection Volume | 10 µL |

The results showed that, as shown in Table 31, when NaTC was incorporated into the CAPE-γCD complex as a super solubilizer, the solubility of CAPE increased in a concentration-dependent manner and it was confirmed that an aqueous solution containing CAPE could be prepared. On the other hand, when NaTC was combined with CAPE as a comparison, a sufficient increase in solubility was not observed.

**[Table 31]**

| CAPE Concentration (µg / mL) | | |
|---|---|---|
| NaTC concentration | 0%(w/v) | 1.0%(w/v) |
| Using a CAPE-γCD Complex | 20 | 237 |
| Using CAPE | 16 | 155 |

### Example 11

### I. Aqueous Solution Containing Propolis

### <Specimen>

### 1) Propolis-γCD Complex

Propolis (Bio30^{®} (23% propolis content, Manauka Health New Zealand) and CAVAMAX® W8 Food (98% or greater γCD, Wacker Chemical Ltd.) were combined to create a preparation.
That is, 130.8 g of Bio30^{®} was measured out after which deionized water (300 mL) and CAVAMAX^{®} W8 Food (79.75 g dry weight) were added and the mixture was mixed at 6,000 rpm for 30 minutes in a homogenizer (IKA ULTRA-TURRAX T25 + Shaft Generator S25KR). After allowing the mixture to stand for 24 hours, the mixture was transferred to petri dishes (ϕ65 mm x 20) and dried overnight via lyophilization (Tokyo Rikakikai Co., Ltd. FD-1000) (dry weight loss: 5.1% (Moisture Analyzer HB43: Mettler Toledo, 105°C)). As a result, a propolis-γCD complex containing 27% (w/w) propolis was obtained as a pale yellow powder.

### 2) Super Solubilizer

A solution created by dissolving polysorbate 80 (Tokyo Chemical Industry Co., Ltd.) in water (polysorbate 80 aqueous solution) was used as a super solubilizer.

### <Preparation of the Aqueous Solution Containing Propolis>

### I - 1. Super Solubilizer: Polysorbate

5 g of 0% (w/v), 0.2% (w/v), 0.5% (w/v), 1% (w/v) or 2% (w/v) polysorbate 80 aqueous solution was added to 12 mg propolis-γCD complex (25% propolis content) and the mixture was stirred (0.08% (w/w) propolis). After the mixture was heated to 85°C, it was then cooled back to room temperature.
The aqueous solution was allowed to stand for 3 hours or 4 days, and the resulting test specimen was placed in a test tube and stirred via shaking for two minutes. The suspension thus obtained was placed in a spectrophotometer cell and absorbance was quantified at 660 nm to obtain turbidity. The results showed that, as shown in Table 32, when polysorbate aqueous solution was incorporated into the propolis-γCD complex as a super solubilizer, the solubility of propolis increased in a concentration-dependent manner and it was confirmed that an aqueous solution containing propolis could be prepared.

**[Table 32]**

| Turbidity (660 nm) | | | | | |
|---|---|---|---|---|---|
| Polysorbate 80 Aqueous Solution Concentration | 0% (w/v) | 0.2% (w/v) | 0.5% (w/v) | 1.0% (w/v) | 2.0% (w/v) |
| After 3 Hours | 1.339 | 0.618 | 0.138 | 0.006 | 0.008 |
| After 4 Days | 1.481 | 0.672 | 0.106 | 0.006 | 0.008 |

### Example 12

### J. Aqueous Solution Containing Loquat Leaf Culture Extract

### <Specimen>

### 1) Loquat Leaf Culture Extract-γCD complex

Loquat leaf culture extract powder (80% triterpene content; provided by JX Nippon Oil & Energy) and CAVAMAX^{®} W8 Food (98% or greater γCD, Wacker Chemical Ltd.) were used to create a preparation.
That is, loquat leaf culture extract powder (1 g) was measured out and ethanol (250 g), deionized water (250 mL) and CAVAMAX^{®} W8 Food (9.0 g dry weight) were added and the mixture was mixed. Next, the mixture was concentrated under reduced pressure at 35°C until the total weight of the mixture was reduced to 200 g. After the mixture was allowed to stand for 1 hour it was transferred to petri dishes (ϕ65 mm x 10) and then dried overnight via lyophilization (Tokyo Rikakikai Co., Ltd. FD-1000). As a result, a loquat leaf culture extract-γCD complex containing 10% loquat leaf culture extract was obtained as a white powder.

### 2) Super Solubilizer

A solution created by dissolving polysorbate 80 (Tokyo Chemical Industry Co., Ltd.) in water (polysorbate 80 aqueous solution) was used as a super solubilizer.

### <Preparation of the Aqueous Solution Containing Loquat Leaf Culture Extract>

### J-1. Super Solubilizer: Polysorbate

5 g of 0% (w/v), 0.2% (w/v), 0.5% (w/v), 1% (w/v) or 2% (w/v) polysorbate 80 aqueous solution was added to 10 mg loquat leaf culture extract-γCD complex (10 % loquat leaf culture extract content) and the mixture was stirred (0.02% (w/w) loquat leaf culture extract). After the mixture was heated to 85°C, it was then cooled back to room temperature.
Furthermore, after standing for 1 day, the test specimen was placed in a test tube and stirred via shaking for 2 minutes. The suspension thus obtained was placed in a spectrophotometer cell and absorbance was quantified at 660 nm to obtain turbidity.
As a comparison, 10 mg of loquat leaf culture extract was used in lieu of loquat leaf culture extract-γCD complex and polysorbate 80 aqueous solution was used as a super solubilizer and a preparation was made as detailed above. The preparation was then allowed to stand.
The results showed that, as shown in Table 33, when polysorbate 80 was incorporated into the loquat leaf culture extract-γCD complex as a super solubilizer, the solubility of loquat leaf culture extract increased in a concentration-dependent manner and it was confirmed that an aqueous solution containing loquat leaf culture extract could be prepared.

**[Table 33]**

| Concentration (660 nm) | | | | | |
|---|---|---|---|---|---|
| Polysorbate 80 Aqueous Solution Concentration | 0%(w/v) | 0.2%(w/v) | 0.5%(w/v) | 1.0%(w/v) | 2.0%(w/v) |
| Using Loquat Leaf Culture Extract-γCD Complex | 0.198 | 0.063 | 0.032 | 0.012 | 0.003 |
| Using Loquat Leaf Culture Extract | 0.082 | 0.078 | 0.073 | 0.064 | 0.068 |

### Example 13

### K. Aqueous Solution Containing Nobiletin

### <Specimen>

### 1) Nobiletin

Biletin (dry weight loss: 8% or less, polymethoxy flavonoids (PMF) (total tangeretin and nobiletin content, excluding water content): 10% or greater, Arkray, Inc.), which is a nobiletin-γCD complex, was used.

### 2) Super Solubilizer

NaTC (Wako Pure Chemical Industries, Ltd.) was used as a super solubilizer.

### <Preparation of the Aqueous Solution Containing Nobiletin>

### K-1. Super Solubilizer: Na Taurocholate (NaTC)

2 mL of a solution prepared by dissolving a super solubilizer (0% (w/v), 0.5% (w/v) NaTC, 1% (w/v) NaTC, 2% (w/v) NaTC or 4% (w/v) NaTC) was added to 30 mg of nobiletin and sonication was performed for several seconds using an ultrasonic apparatus (Ikeda Scientific Co., Ltd.).
Next, the mixture was immediately filtered using a 0.2 µm filter and the nobiletin concentration of the resulting solution was analyzed using an HPLC analyzer (Shimadzu, LC-2010C) under the conditions shown in Table 34 to obtain the solubility of nobiletin.

**[Table 34]**

| HPLC Analysis Conditions | |
|---|---|
| Column | Phenomenex Luna 5u C18 (2) 100A (4.6 mm I.D. x 150 mm) |
| Mobile Phase | MeOH : 10 mM Phosphate = 75 : 25 |
| Detector | UV 340 nm |
| Flow Rate | 0.7 mL / min |
| Column Temp | 40°C |
| Injection Volume | 2 µL |

The results showed that, as shown in Table 35, when NaTC was incorporated into the nobiletin-γCD complex as a super solubilizer, the solubility of nobiletin increased in a concentration-dependent manner and it was confirmed that an aqueous solution containing nobiletin could be prepared.

**[Table 35]**

| NaTC concentration (%) | 0% (w/v) | 0.5% (w/v) | 1% (w/v) | 2% (w/v) | 4% (w/v) |
|---|---|---|---|---|---|
| Peak Area (mAU·S) | 6980040 | 7886860 | 8794984 | 9502239 | 13763064 |

### Example 14

### L. Aqueous Solution Containing α-Tocopherol and γ-Tocotrienol

### <Specimen>

### 1) Tocotrienol-Containing Oil-γCD Complex

Tocotrienol (Oryza Tocotrienol^{®}-70 (total tocotrienol and tocopherol content: 70% (including total tocotrienol content of 40% or greater (including 14% or greater α-tocotrienol, and 24% or greater γ-tocotrienol)), Oryza Yuka Ltd.) and CAVAMAX^{®} W8 Food (98% or greater γCD, Wacker Chemical Ltd.) were used to create a preparation.
That is, 45 g of Oryza Tocotrienol^{®}-70 was measured out, CAVAMAX^{®} W8 Food (93.46 g dry weight) and deionized water (257 mL) were added and the mixture was mixed at 10,000 rpm for 30 minutes in a homogenizer (IKA ULTRA-TURRAX T25 + Shaft Generator S25KR) while being heated to 80°C. Deionized water was added as appropriate as viscosity increased.
After allowing the mixture to stand for 24 hours, the mixture was transferred to petri dishes (ϕ65 mm x 20) and then dried via lyophilization (Tokyo Rikakikai Co., Ltd. FD-1000) (dry weight loss: 4.55% (Moisture Analyzer HB43: Mettler Toledo, 120°C)). As a result, tocotrienol-containing oil-γCD complex containing 32.5% Oryza Tocotrienol^{®}-70 was obtained as a milky white powder.

### 2) Super Solubilizer

NaTC (Wako Pure Chemical Industries, Ltd.) or GZK₂ (97.3% or greater content, Tokiwa Phytochemical Co., Ltd.) was used as a super solubilizer.

### <Preparation of the Aqueous Solution Containing α-Tocopherol and γ-Tocotrienol>

### L-1. Super Solubilizer: Sodium Taurocholate (NaTC)

2 mL of a solution formed by dissolving NaTC (0, 0.5% (w/v), 1.0% (w/v) or 2.0% (w/v)) was added to 50 mg of tocotrienol-containing oil-γCD complex (32.5% oil content) and after the mixture was mixed by vigorous shaking, it was left to stand at room temperature for 1 hour. The mixture was then filtered using a 0.2 µm filter and precisely 1 mL of filtrate was taken and lyophilized. After the powder thus obtained was nearly entirely dissolved in 0.1 mL of DMSO and 0.1 mL of deionized water, an extraction was performed with 1 mL of hexane. The concentration of α-tocopherol and γ-tocotrienol contained in the hexane phase (µg / mL) was measured via HPLC (Shimadzu, LC-2010C) under the conditions shown in Table 36 and the solubility of α-tocopherol and γ-tocotrienol was obtained.

As a comparison, 16.3 mg of Oryza Tocotrienol^{®}-70 (in lieu of tocotrienol-containing oil-γCD complex) and a solution prepared by dissolving NaTC (0, 0.5% (w/v), 1.0% (w/v) or 2.0% (w/v)) as a super solubilizer were combined and the mixture was prepared as described above. The resulting mixture was left to stand and filtered and the resulting solution was analyzed using HPLC.

**[Table 36]**

| HPLC Analysis Conditions | |
|---|---|
| Column | SSCPAK PEGASIL Silica SP100 (4.6 mm I.D. x 250 mm) |
| Mobile Phase | Hexane/Dioxane/IPA = 496/7.9/3 (w/w/w) |
| Detector | UV 295 nm |
| Flow Rate | 1.0 mL / min |
| Column Temp | 40°C |
| Injection Volume | 10 µL |

The results showed that, as shown in Table 37, when Na taurocholate aqueous solution is incorporated into tocotrienol-containing oil-γCD complex as a super solubilizer, the solubility of α-tocopherol and γ-tocotrienol increases in a concentration-dependent manner and it was confirmed that an aqueous solution containing α-tocopherol and γ-tocotrienol could be prepared.
On the other hand, when NaTC was combined with Oryza Tocotrienol^{®}-70 as a comparison, a sufficient increase in solubility was not observed.

**[Table 37]**

| Concentration of α-Tocopherol and γ-Tocotrienol (µg / mL) | | | | | |
|---|---|---|---|---|---|
| | NaTC concentration | 0% (w/v) | 0.5% (w/v) | 1.0% (w/v) | 2.0% (w/v) |
| Using the Tocotrienol-Containing Oil-γCD Complex | α-Tocopherol | nd | 3.3 | 6.0 | 99. 9 |
| | γ-Tocotrienol | nd | 4.5 | 34.2 | 517.1 |
| Using Oryza Tocotrienol^{®} -70 | α-Tocopherol | nd | nd | nd | 25.8 |
| | γ-Tocotrienol | nd | nd | 2.6 | 80.0 |

### L-2. Super Solubilizer: GZK₂

2 mL of a solution formed by dissolving super solubilizer (0, 1.0% (w/v), 1.4% (w/v), 1.7% (w/v) or 2.0% (w/v)) was added to 50 mg of tocotrienol-containing oil-γCD complex (32.5% oil content) and after the mixture was mixed by vigorous shaking, it was left to stand at room temperature for 1 hour. The mixture was then filtered using a 0.2 µm filter and precisely 1 mL of filtrate was taken and lyophilized. After the powder thus obtained was nearly entirely dissolved in 0.1 mL of DMSO and 0.1 mL of deionized water, an extraction was performed with 1 mL of hexane. The concentration of α-tocopherol and γ-tocotrienol contained in the hexane phase (µg / mL) was measured via HPLC (Shimadzu, LC-2010C) under the conditions shown in Table 36 and the solubility of α-tocopherol and γ-tocotrienol was obtained.

As a comparison, 16.3 mg of Oryza Tocotrienol^{®}-70 (in lieu of tocotrienol-containing oil-γCD complex) and a solution prepared by dissolving super solubilizer (0, 1.0% (w/v), 1.4% (w/v), 1.7% (w/v) or 2.0% (w/v)) were combined and the mixture was prepared as described above. The resulting mixture was left to stand and filtered and the resulting solution was analyzed using HPLC.

The results showed that, as shown in Table 38, when GZK₂ aqueous solution is incorporated into tocotrienol-containing oil-γCD complex as a super solubilizer, the solubility of α-tocopherol and γ-tocotrienol increases in a concentration-dependent manner and it was confirmed that an aqueous solution containing α-tocopherol and/or γ-tocotrienol could be prepared. On the other hand, when GZK₂ was combined with Oryza Tocotrienol^{®}-70 as a comparison, a sufficient increase in solubility was not observed.

**[Table 38]**

| Concentration of α-Tocopherol and γ-Tocotrienol (µg / mL) | | | | | | |
|---|---|---|---|---|---|---|
| | Concentration of GZK₂ | 0% (w/v) | 1.0% (w/v) | 1.4% (w/v) | 1.7% (w/v) | 2.0% (w/v) |
| Using the Tocotrienol-Containing Oil-γCD Complex | α-Tocopherol | nd | nd | 9.7 | 90.9 | 339.6 |
| | γ-Tocotrienol | nd | nd | 70.7 | 509.7 | 1964.4 |
| Using Oryza Tocotrienol^{®}-70 | α-Tocopherol | nd | nd | nd | nd | nd |
| | γ-Tocotrienol | nd | nd | nd | nd | nd |

### Example 15

### <Confirmation Testing>

The incorporation of an aqueous solution containing a high concentration of tocotrienol, which was obtained in the same manner as Example 14, into skin was checked.

### <Specimen>

### 1) Test Specimen

Test specimen: 33 mg of GZK₂ was mixed into 60 mg of tocotrienol-containing oil-γCD complex (containing 19.5 mg of tocotrienol-containing oil) and the resulting mixture was stirred after adding 3 mL of ultra-pure water. The resulting mixture was then filtered using a 0.2 µm filter and the filtrate was used as a test specimen.

### 2) Comparative Specimen

Comparative Specimen 1: 3 mL of ultra-pure water was added to 10 mg of tocotrienol-containing oil-γCD complex (32.5% oil content) and sonicated for 30 minutes. The resulting mixture was used as a comparative specimen (tocotrienol-containing oil-γCD complex suspension).

### <Skin Incorporation Testing>

The "LabCyte EPI-MODEL 12" 3-dimensional cultured human skin model (Japan Tissue Engineering Co., Ltd.) was used to evaluate the incorporation of α-tocopherol and γ-tocotrienol into the skin (dermal absorption).
That is, after incubating (37°C, 5% CO₂) the 3-dimensional cultured human skin model overnight in assay medium, the model was exposed to the 200 µL of the aforementioned test specimen or comparative specimen and incubated (37°C, 5% CO₂) for 6 hours.
Next, the exposed test specimen or comparative specimen was washed with 0.1 M PBS (pH 7.2) and the α-tocopherol and γ-tocotrienol contained in the 3-dimensional cultured human skin model was extracted with chloroform / methanol (1:1) and quantified using LL-MS under the conditions shown in Table 39.
The results showed that, as shown in Table 40, the test specimen prepared by combining tocotrienol-containing oil-γCD complex with GZK₂ as a super solubilizer was incorporated into the skin (showed dermal absorption) at a rate approximately 4 times that of the comparative specimen, constituting a significant increase.

**[Table 39]**

| | |
|---|---|
| Column | Phenomenex ; Luna 5u C18(2) 100A (4.6 mm I.D. x 150 mm) |
| Mobile Phase | 2.5 mM acetate buffer : MeOH = 0.1 : 100 |
| Flow Rate | 0.2 mL/min |
| Temperature | 35°C |
| Detection | MS; ESI (+) m/z 429, 409, ([M-H]⁻) |
| Injection Volume | 20 µL |

**[Table 40]**

| Concentration of α-Tocopherol and γ-Tocotrienol (µg / well) | | | |
|---|---|---|---|
| Test Specimen | Tocotrienol-containing oil-γCD complex + GZK₂ solution | α-Tocopherol | 0.14 |
| | | γ-Tocotrienol | 1.79 |
| Test Specimen 1 | Tocotrienol-containing oil-γCD complex suspension | α-Tocopherol | 0.04 |
| | | γ-Tocotrienol | 0.43 |

### Example 16

### Preparation of a Cosmetic Product

### 1. Preparation of the Components

### 1) Preparation of a Cosmetic Toner or Lotion Containing Super Solubilizer

A cosmetic toner containing GZK₂ as a super solubilizer was prepared using the composition specified in Table 41. Furthermore, a lotion containing GZK₂ as a super solubilizer was prepared using the composition specified in Table 42. Note also that commonly-used antioxidant preservatives and perfuming agents were selected as appropriate.

**[Table 41]**

| Cosmetic Toner | |
|---|---|
| Ingredients | % Amount (w/v) |
| Ethanol | 5.00 |
| Glycerin | 2.00 |
| 1,3-butylene glycol | 2.00 |
| Polyethylene oleyl ether | 0.50 |
| Sodium citrate | 0.10 |
| Citric acid | 0.10 |
| GZK₂ | 0.50 |
| Preservative | As Needed |
| Perfume agent | As Needed |
| Purified water | Remainder |
| Total Amount | 100.00 |

**[Table 42]**

| Lotion | |
|---|---|
| Ingredients | % Amount (w/v) |
| Stearic Acid | 2.00 |
| Cetyl alcohol | 2.00 |
| Lanolin alcohol | 2.00 |
| Liquid paraffin | 7.00 |
| Cyclomethicone | 2.00 |
| Polyoxyethylene monooleate | 0.10 |
| Glycerin | 3.00 |
| Propylene glycol | 5.00 |
| Triethylamine | 1.00 |
| Sodium polyacrylate | 0.15 |
| GZK₂ | 0.50 |
| Preservative | As Needed |
| Perfume agent | As Needed |
| Purified water | Remainder |
| Total Amount | 100.00 |

2) Preparation of a Cream Containing a Fat-Soluble Substance and CD A cream containing CoQ10-γCD complex was prepared using CAVAMAX^{®} W8 CoQ10 (21.3% CoQ10 content, Wacker Chemical Ltd.) as a source of fat-soluble substance and CD using the composition specified in Table 43.

**[Table 43]**

| Cream | |
|---|---|
| Ingredients | % Amount (w/v) |
| Squalene | 20.00 |
| Beeswax | 5.00 |
| Purified jojoba oil | 5.00 |
| Glycerin | 5.00 |
| Glycerin monostearate | 2.00 |
| Polyoxyethylene (20) sorbitan monostearate | 2.00 |
| Coenzyme Q10-γCD complex | 0.15 |
| Preservative | As Needed |
| Perfume agent | As Needed |
| Purified water | Remainder |
| Total Amount | 100.00 |

### Use as a Cosmetic Product

1) After rubbing 2 - 5 mL of cosmetic toner containing super solubilizer which was prepared in 1.1) above on skin, 1 - 3 g of cream containing fat-soluble substance and CD which was prepared in 1.2) above was applied on top of the toner and the two were mixed together on the skin for use as a cosmetic product. 2) After rubbing 2 - 5 mL of lotion containing super solubilizer which was prepared in 1.1) above on skin, 1 - 3 g of cream containing fat-soluble substance and CD which was prepared in 1.2) above was applied on top of the toner and the two were mixed together on the skin for use as a cosmetic product.

### Industrial Applicability

The production method constituted by the present invention enables the easy provision of an aqueous solution containing a high concentration of fat-soluble substance which is useful in human applications, such as curcumin, CoQ10 and α-lipoic acid. Moreover, the production method constituted by the present invention enables the easy provision of an aqueous solution containing a high concentration of fat-soluble substance which features increased absorption by the skin. As a result, the present invention enables the easy provision of cosmetics, food products and pharmaceutical products which use as a raw material aqueous solutions containing useful fat-soluble substances at high concentrations.

## Claims

1. A method for the production of The aqueous solution containing one or more of the following fat-soluble substances: polyphenols, flavonoids, tocopherols, retinols, menaquinones, carotenoids, propolis or cinnamic acid derivatives, diterpenes, terpenes, coenzyme Q10 or α-lipoic acid, which includes Process A or B below:
A. a process in which one or more of the super solubilizers listed in 1) - 9), cyclodextrin and the aforementioned fat-soluble substance are added to water
B. a process in which one or more of the super solubilizers listed in 1) - 9) and a cyclodextrin complex containing a fat-soluble substance are added to water
1) an alkali salt or bile acid selected from any one of the following: taurocholate, glycocholate, cholic acid, lithocholic acid or deoxycholic acid
2) glycosides of polyphenols or hesperidin
3) an emulsifier selected from any one of the following: glycerin fatty acid ester, sorbitan fatty acid ester, propylene glycol fatty acid ester, or sucrose fatty acid ester
4) a lecithin agent selected from any one of the following: enzymatically decomposed lecithin, lecithin derived from soybean or lecithin derived from egg yolk
5) a saponin selected from any one of the following: glycyrrhizic acid or an alkali salt thereof, soybean saponin or quillaja saponin
6) a casein or alkali salt thereof
7) a polysorbate (fatty acid polyoxyethylene sorbitan)
8) a cellulose or alkali salt thereof selected from any one of the following: methylcellulose, carboxymethyl cellulose or hydroxyethyl cellulose
9) a polysaccharide thickener selected from any one of the following: pectin, carrogeenan or xanthan gum

2. The method specified in claim 1 above in which the cyclodextrin employed is either α-cyclodextrin, β-cyclodextrin, γ-cyclodextrin or branched cyclodextrin.

3. The method specified in claims 1 or 2 above in which the fat-soluble substance is one of those listed in 1) - 11) below:
1) a polyphenol selected from any one of the following: curcumin, rose polyphenol, ferulic acid, quercetin or cacao mass
2) a flavanoid selected from any one of the following: isoflavone, isoflavone aglycone, flavanone, anthocyanidin, catechin, chalcone or nobiletin
3) a tocopherol selected from any one of the following: tocotrienol, γ-tocotrienol, tocopherol, α-tocopherol, tocopherol acetate, tocopheryl linoleic acid or tocopheryl nicotinate
4) a retinol selected from any one of the following: retinol, retinyl acetate or retinyl palmitate
5) a menaquinone selected from any one of the following: phylloquinone, menaquinone or menadione
6) a carotenoid selected from any one of the following: astaxanthin, lutein, fucoxanthin or lycopene
7) propolis or a cinnamic acid derivative selected from any one of the following: caffeic acid phenethyl ester, artepillin C or coumaric acid
8) a diterpene selected from any one of the following: phytol, abietic acid, levopimaric acid or gibberellin
9) a triterpene selected from any one of the following: squalene, lanosterol, cucurbitacin, limonin or loquat leaf culture extract
10) Coenzyme Q10
11) α-lipoic acid.

4. An aqueous solution containing a fat-soluble substance which is produced using one of the methods specified in claims 1 - 3 above.

5. The aqueous solution specified in claim 4 above which is **characterized by** increased absorbance of the fat-soluble substance by the skin.

6. The aqueous solution specified in either claim 4 or 5 above which contains 9 µg / mL or greater curcumin.

7. The aqueous solution specified in either claim 4 or 5 above which contains 5 µg / mL or greater coenzyme Q10.

8. The aqueous solution specified in either claim 4 or 5 above which contains 0.8 mg / mL or greater α-lipoic acid.

9. The aqueous solution specified in either claim 4 or 5 above which contains 5.0 µg / mL or greater retinol.

10. The aqueous solution specified in either claim 4 or 5 above which contains 1.0 mg / mL or greater ferulic acid.

11. The aqueous solution specified in either claim 4 or 5 above which contains 0.01% (w/v) or greater isoflavone.

12. The aqueous solution specified in either claim 4 or 5 above which contains 0.1% (w/w) or greater rose polyphenol.

13. The aqueous solution specified in either claim 4 or 5 above which contains 150 µg / mL or greater caffeic acid phenethyl ester.

14. The aqueous solution specified in either claim 4 or 5 above which contains 0.05% (w/w) or greater propolis.

15. The aqueous solution specified in either claim 4 or 5 above which contains 0.01% (w/w) or greater loquat leaf culture extract.

16. The aqueous solution specified in either claim 4 or 5 above which contains 19 mg / mL or greater nobiletin.

17. The aqueous solution specified in either claim 4 or 5 above which contains 3.3 µg / mL or greater α-tocopherol and / or 4.5 µg / mL or greater γ-tocotrienol.

18. A cosmetic manufactured using the aqueous solution specified in Claims 4 - 17 above as an ingredient.

19. A food product manufactured using the aqueous solution specified in Claims 4 - 17 above as an ingredient.

20. A pharmaceutical product manufactured using the aqueous solution specified in Claims 4 - 17 above as an ingredient.
